(19) Europäisches Patentamt / European Patent Office / Office européen des brevets

(11) **EP 3 688 486 B1**

(12) **EUROPÄISCHE PATENTSCHRIFT**

(45) Veröffentlichungstag und Bekanntmachung des Hinweises auf die Patenterteilung:
**12.03.2025 Patentblatt 2025/11**

(21) Anmeldenummer: **18778896.3**

(22) Anmeldetag: **26.09.2018**

(51) Internationale Patentklassifikation (IPC):
**G01S 7/52** (2006.01)    **G01S 15/89** (2006.01)
**B06B 1/06** (2006.01)    **A61B 8/00** (2006.01)
**A61B 8/13** (2006.01)    **A61B 8/08** (2006.01)
**B06B 1/02** (2006.01)

(52) Gemeinsame Patentklassifikation (CPC):
**G01S 7/52025; A61B 8/13; B06B 1/0215;
G01S 7/52046; G01S 15/8915;** A61B 8/0825;
A61B 8/406; B06B 2201/55

(86) Internationale Anmeldenummer:
**PCT/EP2018/076079**

(87) Internationale Veröffentlichungsnummer:
**WO 2019/063595 (04.04.2019 Gazette 2019/14)**

(54) **VORRICHTUNG ZUR ANSTEUERUNG UND AUSLESE EINER GRUPPE VON ULTRASCHALLWANDLERN FÜR ULTRASCHALL-COMPUTERTOMOGRAPHIE UND ULTRASCHALL-COMPUTERTOMOGRAPH**

DEVICE FOR ACTUATING AND READING A GROUP OF ULTRASOUND TRANSDUCERS FOR ULTRASOUND COMPUTER TOMOGRAPHY, AND ULTRASOUND COMPUTER TOMOGRAPHY MACHINE

DISPOSITIF POUR COMMANDER ET LIRE UN GROUPE DE TRANSDUCTEURS À ULTRASONS POUR RÉALISER UNE ÉCHOTOMOGRAPHIE ET ÉCHOTOMOGRAPHE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **27.09.2017 DE 102017217214**

(43) Veröffentlichungstag der Anmeldung:
**05.08.2020 Patentblatt 2020/32**

(73) Patentinhaber: **Karlsruher Institut für Technologie**
**76131 Karlsruhe (DE)**

(72) Erfinder:
• **PERIC, Ivan**
**76464 Bruchsal (DE)**

• **ZAPF, Michael**
**76149 Karlsruhe (DE)**
• **GEMMEKE, Hartmut**
**76297 Stutensee (DE)**
• **LEYS, Richard**
**66111 Saarbrücken (DE)**

(74) Vertreter: **Altmann Stößel Dick Patentanwälte PartG mbB**
**Theodor-Heuss-Anlage 2**
**68165 Mannheim (DE)**

(56) Entgegenhaltungen:
WO-A1-02/30288        WO-A1-2015/186193
WO-A1-2017/066564    KR-A- 20160 021 354
US-A1- 2016 242 739

**Beschreibung**

Gebiet der Erfindung

Die vorliegende Erfindung betrifft eine Vorrichtung zur Ansteuerung und Auslese einer Gruppe von Ultraschallwandlern für Ultraschall-Computertomographie und einen Ultraschall-Computertomographen, der mindestens eine derartige Vorrichtung umfasst. Der Ultraschall-Computertomograph eignet sich insbesondere für einen Einsatz in ultraschallbasierten Bildgebungsverfahren für die Brustkrebsfrüherkennung. Weitere Einsatzzwecke, insbesondere in der zerstörungsfreie Materialprüfung oder der Testtechnik, sind ebenfalls möglich.

Stand der Technik

Insbesondere für medizinische Untersuchungen einsetzbare Ultraschall-Computertomographen umfassen mindestens einen Ultraschallkopf, mindestens einen Ultraschallwandlern und mindestens eine Steuer- und Auswerteeinheit aufweist, welche Steuersignale für den Ultraschallwandler aussendet und an dem Ultraschallwandler empfangene Messsignale als elektrische Signale aufnimmt, verstärkt und an eine Steuer- und Auswerteeinheit weiterleitet, insbesondere zu einer Rekonstruktion von Echtzeit-Abbildungen auf einem Bildschirm während einer Messung.

WO 2002/030288 A1 offenbart einen Ultraschall-Computertomographen, welcher insbesondere in einem ultraschallbasierten Bildgebungsverfahren für BrustkrebsFrüherkennung einsetzbar ist. Der Ultraschall-Computertomograph arbeitet nach dem Transmissions-Streuungs- und Impuls-Echo-Verfahren und umfasst einen nach oben offenen Behälter, in welchen der zu untersuchende Körperteil eingeführt wird, Ultraschallwandler, die an einer Behälterwandung über die gesamte Wandungsflache fest angeordnet sind und deren Hauptabstrahlrichtung jeweils senkrecht von der Wandungsflache in das Behälterinnere ausgerichtet ist, ein Ankopplungsmedium, welches in dem Behälter eingefüllt den zu untersuchenden Körperteil benetzt und zur Ankopplung und Übertragung von Ultraschallsignalen zwischen den Ultraschallwandlern und dem zu untersuchenden Körperteil dient, sowie eine rechnergestützte Steuer- und Auswerteeinheit mit Arbeitsspeicher, welcher mit den Ultraschallwandlern verschaltet ist. Die Verschaltung wird hierbei derart ausgeführt, das eine beliebige Anzahl der Ultraschallwandler sowohl als Sender als auch als Empfänger über einen elektronischen Schalter anwählbar sind, dass die von den Sendern ausgesendeten Ultraschallsignale Ultraschallimpulse sind und die von allen Empfängern parallel empfangenen Signale als elektrische Signale verstärkt, gefiltert, digitalisiert und im Arbeitsspeicher als Daten abgespeichert werden, dass aus den im Arbeitsspeicher abgelegten Daten Schalllaufzeiten und über diese und die geometrischen Verhältnisse einzelne Schallgeschwindigkeiten ermittelt sowie durch eine rechnerische Aufteilung des Behältervolumens in zahlreiche Bereiche sowie durch eine Korrelation verschiedener Datensätze die Schallgeschwindigkeiten in den einzelnen Bereichen berechnet werden, dass aus den Schallgeschwindigkeiten in den einzelnen Bereichen sowie aus Amplitude und Phasenverlauf der empfangenen Signale alle möglichen Reflexionspunkte in dem Behälter berechnet werden sowie die Signale als Daten für die möglichen Reflexionspunkte aus allen Messungen für jeden Punkt im Behälter aufsummiert werden, hieraus für jeden Punkt eine der Höhe des Summenwertes entsprechender Farbwert zugeordnet und diese je nach gewünschter Auflösung je mindestens einem Pixel in der dreidimensionalen Rekonstruktion zugeordnet wird.

Details zu weiteren Auswertungs- und Rekonstruktionsverfahren, welche hier ebenfalls eingesetzt werden können, finden sich in darüber hinaus in EP 2 056 124 A1, WO 2011/124379 A2 und WO 2012/110228 A1.

Eine der Herausforderungen in Ultraschall-Computertomographen besteht in einer Ausgestaltung einer geeigneten Vorrichtung zur Ansteuerung und Auslese von einem oder mehreren Ultraschallwandlern. Diese Vorrichtung muss insbesondere dazu eingerichtet sein, um sowohl Hochspannungssignale, die zur Erzeugung eines ausreichenden akustischen Drucks erforderlich sind, verarbeiten zu können als auch schwache Antwortsignale verstärken zu können. Hierzu umfasst die Vorrichtung üblicherweise eine Hochfrequenz-Pulseinheit, einen Hochfrequenz-Isolationsschalter und einen rauscharmen Niederspannungs-Vorverstärker.

WO 2005/107962 A1 offenbart Ultraschallwandler mit Wandlerarrays, welche eine Anzahl von Wandlerelementen aufweisen. Jeder Ultraschallwandler umfasst hierbei mindestens einen piezoelektrischen Körper, der jeweils mindestens eine untere und eine obere Elektrode sowie eine Ankopplungsschicht aufweist, welche mindestens eine Platine umfasst, auf die der Ultraschallwandler mit der unteren Elektrode verbunden ist, wobei auf den Platinen Leiterbahnstrukturen mit konstanter Leiterbahnhöhe unter den piezoelektrischen Köpern vorgesehen sind, welche in einem elektrischen Kontakt mit der unteren Elektrode stehen, und wobei ein verbleibender Hohlraum zwischen der Platine und dem piezoelektrischen Körper neben der Leiterbahnstruktur vollständig durch ein Bindemittel ausgefüllt ist.

US 2001/0043090 A1 offenbart einen integrierten Schaltkreis als Vorrichtung zur Ansteuerung und Auslese eines Ultraschallwandlers. Der monolithisch auf einem einzigen Substrat angeordnete Schaltkreis umfasst hierbei sowohl einen Niederspannungsschaltkreis als auch einen Hochspannungsschaltkreis, wobei der Niederspannungsschaltkreis auf Basis von CMOS aufgebaut ist, während der Hochspannungsschaltkreis über einen Hochspannungs-FET verfügt. Der Niederspannungsschaltkreis weist hierbei einerseits einen digitalen logischen Schaltkreis auf, welcher dazu eingerichtet ist, um Signale zu erzeugen und zu einem Ultraschallwandlerelement zu übertragen, und andererseits einen analogen Schaltkreis, um Signale des Ultraschallwandlerelements zu empfangen und weiterzuverarbeiten, während der Hochspannungsschaltkreis zur Ansteuerung des Ultraschallwandlerelements dient.

Vergleichbare Vorrichtungen finden sich in US 2009/0146695 A1 und US 2016/0242739 A1, in welchen Pulse zur Ansteuerung des Ultraschallwandlerelements verwendet werden.

US 2015/0032002 A1 offenbart eine Ultraschallwandlersonde mit einer Mehrzahl an Ultraschallwandlerelementen und zugehörigen Schaltungen. Hierbei wird vorgeschlagen, die Sendeverstärker aus CMOS-Bauteilen aufzubauen. Ferner wird durch die Verwendung einer N-Wanne in dem Transistor-Aufbau eine Isolation von dem Substrat erreicht. Weiterhin ist ein Ausgansschalter vorgesehen, welcher dazu eingerichtet ist, um zumindest während der Sendepause eine Beaufschlagung eines Niederspannungsvorverstärkers mit dem Messsignal zu unterbinden. Ferner werden Multiplexer-Schaltungen offenbart.

D.F. Lemmerhirt, X. Cheng, R.D. White, C.A. Rich, M. Zhang, J.B. Fowlkes und O.D. Kripfgans, A 32 × 32 Capacitive Micromachined Ultrasonic Transducer Array Manufactured in Standard CMOS, IEEE Transactions on Ultrasonics, Ferroelectrics, and Frequency Control, Vol. 59, No. 7, 2012, beschreibt einen Prototypen eines Wandlerarrays, in welchem der Ultraschallwandler und der zu dessen Ansteuerung und Auslese eingerichtete Schaltkreis ebenfalls auf einem gemeinsamen Substrat integriert sind. Hierbei beaufschlagen Pulse die untere Platte eines jeden kapazitiven mikromechanisch hergestellten Ultraschallwandlers (engl. *Capacitive Micromachined Ultrasonic Transducer,* kurz CMUT), wobei ein Schalter in einer AN-Position den Eingang einer CMOS Elektronik schützt und in einer AUS-Position einen Ausgangsstrom aus dem CMUT in einen Spannungswert umwandelt.

G. Gurun, M.S. Qureshi, M. Balantekin, R. Guldiken, J. Zahorian, S.-Y. Peng, A. Basu, M. Karaman, P. Hasler und L. Degertekin, Front-end CMOS Electronics for Monolithic Integration with CMUT Arrays: Circuit Design and Initial Experimental Results, 2008 IEEE International Ultrasonics Symposium Proceedings (ULTSYM) 0096; I. Cicek, A. Bozkurt und M. Karaman, Design of a Front-End Integrated Circuit for 3D Acoustic Imaging Using 2D CMUT Arrays, IEEE Transactions on Ultrasonics, Ferroelectrics, And Frequency Control, Vol. 52, No. 12, 2005; J. Song, S. Jung, Y. Kim, K. Cho, B. Kim, S. Lee, J. Nab, I. Yang, O.-K. Kwon und D. Kim, Reconfigurable 2D cMUT-ASIC Arrays for 3D Ultrasound Image, Proc. of SPIE Vol. 8320 83201A-1; S.-J. Jung, et al., Three-Side buttable integrated ultrasound chip with a 16x16 reconfigurable transceiver and capacitive micromachined ultrasonic transducer array for 3-D ultrasound imaging systems, IEEE Trans. Electron Dev., Vol. 60, No.10, S. 3562-3569, 2013; R. Wodnicki, Modular ultrasound arrays with co-integrated electronics, Proc. of the 14th International Symposium on Nondestructive Characterization of Materials, p. 23, 2015 sowie H.-K. Cha, CMOS ultrasonic analogue front-end with reconfigurable pulser/switch for medical imaging applications, Electronics Letters Vol. 51 No. 20, S. 1564-1566, 2015 stellen weitere Vorrichtungen vor, in welchen Ultraschallwandler sowie zu deren Ansteuerung und Auslese eingerichtete Schaltkreise monolithisch auf einem gemeinsamen Substrat integriert sind, wobei jedoch nur der Niederspannungsschaltkreis auf Basis von CMOS aufgebaut ist.

Im Gegensatz hierzu sind in H. Kuruveettil, D. Zhao, C.J. Hao und M. Je, Analog Front End Low Noise Amplifier in 0.18-$\mu$m CMOS for Ultrasound Imaging Applications, International Journal of Electrical, Computer, Energetic, Electronic and Communication Engineering, Vol. 7, No. 9, 2013, der Ultraschallwandler und ein zur Signalauslese eingerichteter Vorverstärker voneinander getrennt auf unterschiedlichen Substraten angeordnet.

Darüber hinaus beschreiben K. Chen, H.-S. Lee, A.P. Chandrakasan und C.G. Sodini, Ultrasonic Imaging Transceiver Design for CMUT: A Three-Level 30-Vpp Pulse-Shaping Pulser With Improved Effciency and a Noise-Optimized Receiver, IEEE Journal Solid-State Circuits, Vol. 48, No. 11, 2013, einen Vierkanal-Empfänger für Ultraschall-Computertomographie, welcher als Schnittstelle für einen mikromechanisch hergestellten Ultraschallwandler (CMUT) dient, sowie einen Hochspannungssender, der über eine dreistufige Pulsformeinheit verfügt. Weiterhin umfasst der Empfänger einen rauscharmen Verstärker, welcher dazu eingerichtet ist, um das schwache Eingangssignal zu verstärken, wobei ein Schalter auf AUS gestellt wird, wodurch verhindert wird, dass Hochspannungstransienten den rauscharmen Verstärker, der mit Niederspannungstransistoren ausgestattet ist, zerstören können.

WO 2017/066564 A1 offenbart ein Verfahren, das folgende Schritte umfasst: Empfangen einer Vielzahl von Analogsignalen einer Vielzahl auf einer Kathetersonde angeordneten Wandlern; Selektives Abtasten der Vielzahl der Analogsignale; Multiplexen zur Erzeugung einer Folge von Abtastwerten; und Übertragen der Folge der Abtastwerte an eine Empfängerschaltung. Die Empfängerschaltung umfasst einen Taktgeber; einen Analog-Digital-Wandler in Verbindung mit dem Taktgeber; und eine Abtastphasenkorrekturschaltung in Verbindung mit dem Taktgeber und dem Wandler. Das Verfahren umfasst ferner: Bestimmen optimaler Abtastzeiten auf der Grundlage gemessener Signalverzögerungen, die mit dem System verbunden sind; Einstellen einer Phase des Taktes auf der Grundlage der gemessenen Signalverzögerungen; und Übertragen des phasenjustierten Taktes an die Senderschaltung für die selektive Abtastung der Analogsignale.

KR 2016 0021354 A offenbart eine Vorrichtung zur Ansteuerung und Auslese einer Gruppe von Ultraschallwandlern für Ultraschall-Computertomographie gemäß dem Oberbegriff des Anspruchs 1. Eine weitere Vorrichtung zur Ansteuerung und Auslese einer Gruppe von Ultraschallwandlern für Ultraschall-Computertomographie wird in WO 2015/186193 A1 beschrieben.

Trotz der Verbesserungen durch die oben genannten Vorrichtungen bleiben noch viele Herausforderungen im Bereich der Ausgestaltung von Vorrichtungen zur Ansteuerung und Auslese einer Gruppe von Ultraschallwandlern in Ultraschall-Computertomographen ungelöst.

Aufgabe der Erfindung

Ausgehend von der Darstellung des Standes der Technik, besteht die Aufgabe der vorliegenden Erfindung darin, eine Vorrichtung zur Ansteuerung und Auslese einer Gruppe von Ultraschallwandlern und einen Ultraschall-Computertomographen bereitzustellen, welche die aufgeführten Nachteile und Einschränkungen des Standes der Technik zumindest teilweise überwinden.

Insbesondere soll die Vorrichtung eine reproduzierbare Sensorcharakteristik sowie trotz kleiner aktiver Sensorfläche ein hohes Signal-Rausch-Verhältnis und eine hohe Signaldynamik bei einem niedrigen Gesamtpreis ermöglichen. Hierbei sollen einerseits Ultraschallempfänger für Signalstärken von μV eingerichtet sein, während andererseits Signalstärken der Ultraschallsender für eine omnidirektionale Abstrahlcharakteristik, eine große Bandbreite sowie nicht-fokussierten Ultraschall 80 V bis 120 V Anregungsspannung erreichen können. Zudem soll der Ultraschallwandler gleichzeitig kleine Reflexionssignale und großen Transmissionssignale registrieren können und die hierzu erforderliche hohe Signaldynamik bereitstellen. Darüber hinaus sollen Ultraschallsender und Ultraschallempfänger möglichst gleiche Bandbreiten und eine hohe Linearität besitzen, um eine gute Rauschanpassung sowie eine große Signaltreue für eine Wiedererkennung der Signale, insbesondere zu einem Nachweis und zu einer Trennung von Transmissions- und Reflexionssignalen, zu ermöglichen.

Weiterhin soll der Ultraschall-Computertomograph über eine mehrerer Gruppen an Ultraschallwandlern verfügen können, um so ein Abtasten des Raumes in drei Dimensionen zu ermöglichen, ohne dass hierfür eine mechanische Bewegung der Ultraschallwandler erforderlich ist. Insbesondere sollen mindestens 100 Vorrichtungen über eine dreidimensionale Messgeometrie verteilt angeordnet sein können, wobei jede Vorrichtung über mindestens 3 Ultraschallwandler, die als Sender und/oder Empfänger fungieren können, verfügen kann, um so eine schnelle Datenaufnahme zu ermöglichen.

Offenbarung der Erfindung

Diese Aufgabe wird durch eine Vorrichtung zur Ansteuerung und Auslese einer Gruppe von Ultraschallwandlern für Ultraschall-Computertomographie und durch einen Ultraschall-Computertomographen gemäß den Merkmalen der unabhängigen Ansprüche gelöst. Vorteilhafte Weiterbildungen, welche einzeln oder in beliebiger Kombination realisierbar sind, sind in den abhängigen Ansprüchen dargestellt.

Im Folgenden werden die Begriffe "haben", "aufweisen", "umfassen" oder "einschließen" oder beliebige grammatikalische Abweichungen davon in nicht-ausschließlicher Weise verwendet. Dementsprechend können sich diese Begriffe sowohl auf Situationen beziehen, in welchen, neben den durch diese Begriffe eingeführten Merkmalen, keine weiteren Merkmale vorhanden sind, oder auf Situationen, in welchen ein oder mehrere weitere Merkmale vorhanden sind. Beispielsweise kann sich der Ausdruck "A hat B", "A weist B auf", "A umfasst B" oder "A schließt B ein" sowohl auf die Situation beziehen, in welcher, abgesehen von B, kein weiteres Element in A vorhanden ist (d.h. auf eine Situation, in welcher A ausschließlich aus B besteht), als auch auf die Situation, in welcher, zusätzlich zu B, ein oder mehrere weitere Elemente in A vorhanden sind, beispielsweise Element C, Elemente C und D oder sogar weitere Elemente.

Weiterhin wird darauf hingewiesen, dass die Begriffe "mindestens ein" und "ein oder mehrere" sowie grammatikalische Abwandlungen dieser Begriffe, wenn diese in Zusammenhang mit einem oder mehreren Elementen oder Merkmalen verwendet werden und ausdrücken sollen, dass das Element oder Merkmal einfach oder mehrfach vorgesehen sein kann, in der Regel lediglich einmalig verwendet werden, beispielsweise bei der erstmaligen Einführung des Merkmals oder Elementes. Bei einer nachfolgenden erneuten Erwähnung des Merkmals oder Elementes wird der entsprechende Begriff "mindestens ein" oder "ein oder mehrere" in der Regel nicht mehr verwendet, ohne dass hierdurch die Möglichkeit eingeschränkt wird, dass das Merkmal oder Element einfach oder mehrfach vorgesehen sein kann.

Weiterhin werden im Folgenden die Begriffe "vorzugsweise", "insbesondere", "beispielsweise" oder ähnliche Begriffe in Verbindung mit optionalen Merkmalen verwendet, ohne dass alternative Ausführungsformen hierdurch beschränkt werden. So sind Merkmale, welche durch diese Begriffe eingeleitet werden, optionale Merkmale, und es ist nicht beabsichtigt, durch diese Merkmale den Schutzumfang der Ansprüche und insbesondere der unabhängigen Ansprüche einzuschränken. So kann die Erfindung, wie der Fachmann erkennen wird, auch unter Verwendung anderer Ausgestaltungen durchgeführt werden. In ähnlicher Weise werden Merkmale, welche durch "in einer Ausführungsform der Erfindung" oder durch "in einem Ausführungsbeispiel der Erfindung" eingeleitet werden, als optionale Merkmale verstanden, ohne dass hierdurch alternative Ausgestaltungen oder der Schutzumfang der unabhängigen Ansprüche eingeschränkt werden soll. Weiterhin sollen durch diese einleitenden Ausdrücke sämtliche Möglichkeiten unangetastet bleiben, die hierdurch eingeleiteten Merkmale mit anderen Merkmalen zu kombinieren, seien es optionale oder nicht-optionale Merkmale.

In einem ersten Aspekt betrifft die vorliegende Erfindung eine Vorrichtung zur Ansteuerung und Auslese einer Gruppe von Ultraschallwandlern für Ultraschall-Computertomographie. Diese Vorrichtung umfasst hierbei

- ein gemeinsames Substrat, wobei das Substrat einen als Eingangskanal ausgestalteten ersten Bereich und einen elektrisch hiervon abgetrennten, als Ausgangskanal ausgestalteten zweiten Bereich aufweist, wobei eine Abtrennung des ersten Bereichs von dem zweiten Bereich mittels n-Wannen erfolgt;
- mindestens einen Hochspannungssignaleingang, welcher auf dem ersten Bereich angeordnet ist, wobei der Hochspannungssignaleingang dazu eingerichtet ist, um analoge Hochspannungssignale aufzunehmen;
- mindestens einen Hochspannungsverstärker, welcher auf dem ersten Bereich angeordnet ist, wobei der Hochspannungsverstärker dazu eingerichtet ist, um die von dem Hochspannungssignaleingang bereitgestellten analogen Hochspannungssignale aufzunehmen und zu verstärken, wobei der Hochspannungsverstärker über Hochvolt-CMOS-Halbleiterbauelemente verfügt;
- mindestens eine Gruppe von Ultraschallwandlern, welche auf dem ersten Bereich angeordnet sind, wobei mindestens einer der Ultraschallwandler als Ultraschallsender eingerichtet ist, um während einer Sendephase, in welcher der Hochspannungsverstärker an seinem Ausgang Hochspannungssignale bereitstellt, Ultraschallsignale erzeugen, und wobei mindestens einer der Ultraschallwandler zur Aufnahme von Ultraschallmesssignalen eingerichtet ist;
- mindestens einen Multiplexer, welcher dazu eingerichtet ist, um Ultraschallmesssignale, welche von mindestens zwei voneinander verschiedenen Ultraschallwandlern aufgenommen wurden, zu einem gemeinsamen Messsignal zu vereinen;
- mindestens einen rauscharmen Niederspannungsvorverstärker, welcher auf dem zweiten Bereich angeordnet ist, wobei der Niederspannungsvorverstärker für einen Empfang des Messsignals eingerichtet ist, wobei der Niederspannungsvorverstärker über CMOS-Halbleiterbauelemente verfügt;
- mindestens einen Ausgangsschalter, welcher auf dem zweiten Bereich angeordnet ist, wobei der Ausgangsschalter dazu eingerichtet ist, um zumindest während der Sendephase eine Beaufschlagung des Niederspannungsvorverstärkers mit dem Messsignal zu unterbinden; und
- mindestens einen Niederspannungssignalausgang, welcher auf dem zweiten Bereich angeordnet ist, welcher zu einer Übertragung eines vorverstärkten Messsignals an eine externe Steuer- und Auswerteeinheit eingerichtet ist.

Die vorliegende Vorrichtung ist vorzugsweise in Form von integrierten Schaltkreisen ausgeführt, insbesondere als mindestens eine anwendungsspezifische integrierte Schaltung (engl. *application-specified integrated circuit;* ASIC). Alternativ könnte die Vorrichtung auch als Universalschaltkreis, insbesondere als FPGA (engl. *field-programmable gate array)* oder als FPAA (engl. *field-programmable analog array*) ausgeführt sein. Weitere Arten der Ausführung der vorliegenden Vorrichtung sind jedoch möglich.

Die vorliegende Vorrichtung ist zur Bereitstellung der integrierten Schaltkreise auf ein gemeinsames Substrat aufgebracht. Das Substrat kann hierbei bevorzugt auf einer Leiterplatte (engl. *printed circuit board,* PCB), welche als Träger für elektronische Bauelemente ausgestaltet ist, aufgebracht sein. Die Leiterplatte kann hierbei einerseits zur mechanischen Stabilität der aufgebrachten elektronischen Bauelemente und andererseits zur Bereitstellung von elektrischen Verbindungen zwischen den elektronischen Bauelementen dienen.

Das für die vorliegende Vorrichtung eingesetzte Substrat verfügt über zwei elektrisch voneinander getrennte Bereiche, wobei eine Abtrennung der beiden Bereiche voneinander mittels n-Wannen erfolgt. Der Begriff der "n-Wanne" bezeichnet hierbei einen abgegrenzten Flächenbereich auf dem Substrat, welcher dazu eingerichtet ist, um Ladungsträger aufzusammeln und einem lateralen Strom entlang des Substrats zu entziehen. Auf diese Weise kann die n-Wanne dazu eingesetzt werden, um elektrisch voneinander getrennte Bereiche auf dem Substrat bereitzustellen. Vorteilhaft an der Abtrennung der beiden Bereiche durch n-Wannen ist es, dass Niederspannungs-Bauelemente auf dem zweiten Bereich vor der auf dem ersten Bereich auftretenden Hochspannung geschützt werden können und dass sich eine Ausbreitung von möglichen Störungen aus dem ersten Bereich in den zweiten Bereich weitgehend unterdrücken lässt.

Im vorliegenden Fall kann auf diese Weise ein als Eingangskanal ausgestalteter ersten Bereich und ein hiervon abgetrennter, als Ausgangskanal ausgestalteter zweiten Bereich geschaffen werden. Während der erste Bereich dazu eingerichtet sein kann, um erste elektronische Bauelemente aufzunehmen, welche zur Aufnahme und Weiterverarbeitung von Hochspannung ausgelegt sind, kann der zweite Bereich dazu eingerichtet sein kann, um zweite elektronische Bauelemente aufzunehmen, welche zur Aufnahme und Weiterverarbeitung von Niederspannung ausgelegt sind. Im Rahmen der vorliegenden Erfindung bezeichnet der Begriff der "Hochspannung" elektrische Spannungen und Potentiale von 5 V bis 120 V, während der der Begriff der "Niederspannung" elektrische Spannungen und Potentiale von 0 V bis 5 V bezeichnet.

Der erste, für die Aufnahme und Weiterverarbeitung von Hochspannung eingerichtete Bereich auf dem Substrat umfasst hierbei mindestens einen Hochspannungssignaleingang, mindestens einen Hochspannungsverstärker und mindestens eine Gruppe von Ultraschallwandlern. Hierbei ist der Hochspannungssignaleingang dazu eingerichtet ist, um analoge Hochspannungssignale aufzunehmen, welche insbesondere von einem externen Hochspannungssender bereitgestellt werden können, und über den Hochspannungsverstärker an die Ultraschallwandler zu führen. Die hierbei verwendeten Hochspannungssignale können insbesondere als Wechselspannung konstanter Amplitude, deren Frequenz periodisch und stetig einen vorgegebenen Bereich durchlaufen kann, vorgegeben werden und daher auch als "Sweep" bezeichnet werden. Andere Arten von Hochspannungssignalen, insbesondere Signalverarbeitungsketten mit codierter Anregung, etwa durch Chirp, Golay- oder Barker Codes, und mit nachgelagerten Korrelationsfiltern zur Erhöhung des Signal-Rausch-Verhältnisses bei hoher Bandbreite, sind jedoch ebenfalls möglich.

Der Hochspannungsverstärker ist dazu eingerichtet ist, um die von dem Hochspannungssignaleingang bereitgestellten analogen Hochspannungssignale aufzunehmen und zu verstärken. Hierzu verfügt der Hochspannungsverstärker über mindestens einen analogen, linearen, rückgekoppelten Verstärker, welcher mindestens drei gesonderte Verstärkerstufen umfasst. Für den Aufbau des Hochspannungsverstärkers sind die elektronischen Bauelemente, insbesondere hierbei verwendete Transistoren, als Hochvolt-CMOS-Halbleiterbauelemente ausgestaltet. Hierfür können vorzugsweise elektronische Bauteile auf Basis einer so genannten 0,35 μm Hochvolt-CMOS-Technologie oder auch 0,18 μm Hochvolt-CMOS-Technologie eingesetzt werden. Während sich der Begriff "CMOS" (engl. *complementary metal-oxide-semiconductor*) auf Halbleiterbauelemente bezieht, in welchen sowohl p-Kanal- als auch n-Kanal-Metall-Oxid-Halbleiter-Feldeffekttransistoren (engl. *metal oxide semiconductor field-effect transistors*; MOSFETs) auf einem gemeinsamen Substrat eingesetzt werden und auch ohne weitere Angaben üblicherweise für einen Betrieb in Niederspannung eingerichtet sind, handelt es sich bei den Hochvolt-CMOS-Halbleiterbauelementen um spezielle Bauelemente, welche sich für den Einsatz bei Hochspannung eignen, insbesondere da sie über eine Spannungsfestigkeit von bis 120V verfügen.

In einer besonders bevorzugten Ausgestaltung verfügen die vorgeschlagene Vorrichtung und die damit umfassten Ultraschallwandler über eine möglichst hohe Bandbreite, um bei einer Verwendung von linearen Bildgebungsverfahren über eine vollständige Abdeckung des räumlichen Frequenzbildraums einen möglichst hohen Kontrast und damit eine möglichst hohe Eindeutigkeit einer Abbildung von komplexen Objekte, wie z.B. eine Brust einer Patientin, erzielen zu können. Darüber hinaus können auch komplexere Bildgebungsverfahren, welche insbesondere auf wellenbasierten Inversionsverfahren basieren können, von niedrigeren Frequenzanteilen profitieren. Vorteilhaft ist zudem ein großer Dynamikhub des Hochspannungsverstärkers, da die für die 3D-Ultraschall-Computertomographie eingesetzten Ultraschallwandler trotz ihrer omnidirektionalen Abstrahlcharakteristik näherungsweise als Punktquellen mit geringer Fläche und niedrigem Abstrahldruck betrachtet werden können. Deswegen umfasst der Hochspannungsverstärker in dieser besonders bevorzugten Ausgestaltung einen linearen, rückgekoppelten Verstärker mit hoher Verstärkung. Dadurch kann eine große relative Bandbreite von mindestens 50 %, bevorzugt mindestens 100 %, besonders bevorzugt mindestens 150 %, erzielt werden. Beispielsweise kann die relative Bandbreite 160 % um eine Mittelfrequenz von 2,5 MHz betragen, was einer Bandbreite von ca. 4 MHz bei 2,5 MHz entspricht.

Um die gewünschte hohe Verstärkung erreichen zu können, umfasst der analoge, lineare, rückgekoppelte Verstärker mindestens drei gesonderte Verstärkerstufen, wobei jeder der Verstärkerstufen jeweils mindestens zwei Hochvolt-CMOS-Transistoren aufweist. In einer bevorzugten Ausgestaltung befindet sich am Eingang einer ersten Verstärkerstufe ein Differentialverstärker, welcher derart ausgestaltet ist, dass eine Differenz von Absolutwerten der Ausgangsströme proportional zur Differenz der an den beiden Eingängen des Differentialverstärkers anliegenden Eingangsspannungen ist. Weiterhin sind die erste Verstärkerstufe und eine zweite Verstärkerstufe jeweils zwischen zwei Stromspiegeln angeordnet. Dieser kann insbesondere ermöglichen, dass ein aus einem Eingang herausfließender Strom ungefähr einem aus einem anderen Ausgang herausfließender entspricht, so dass die erste und die zweite Verstärkerstufe unabhängig voneinander jeweils mit denselben Strömen beaufschlagt werden können. Weiterhin sind in der zweiten Verstärkerstufe und in einer dritten Verstärkerstufe die Hochvolt-CMOS-Transistoren jeweils über ihre Sources miteinander verbunden, insbesondere um zu ermöglichen, dass an den Ausgang des Hochspannungsverstärkers ein niedriger Widerstand und/oder eine hohe Kapazität angeschlossen werden können, ohne die Hochspannungsverstärkung signifikant verringert wird. Der beschriebene Aufbau des analogen, linearen, rückgekoppelten Verstärkers ermöglicht eine konstante, lineare Verstärkung für die verwendeten hohen Spannungen bei einer Spannungsfestigkeit von mindestens 80 V, bevorzugt von mindestens 120 V.

Wie bereits erwähnt, ist weiterhin mindestens eine Gruppe von Ultraschallwandlern ebenfalls auf dem ersten, als Eingangskanal ausgestalteten Bereich angeordnet. Hierbei kann die Gruppe von Ultraschallwandlern eine Mehrzahl von Ultraschallwandlern verfügen, insbesondere 2, 3, 4, 5, 6, 8, 9, 10, 12, 13, 15, 16, 18, 20, 24, 25 oder mehr Ultraschallwandler. Eine andere Anzahl von Ultraschallwandlern ist jedoch möglich. Der Begriff des "Ultraschallwandlers" bezeichnet hierbei ein elektronisches Bauelement, welches dazu eingerichtet ist, um eine elektrische Größe, insbesondere einen elektrischen Strom oder, vorzugsweise, eine elektrische Spannung, in Ultraschallsignale oder umgekehrt Ultraschallsignale in eine elektrische Größe, insbesondere einen elektrischen Strom oder, vorzugsweise, in eine elektrische Spannung, umzuwandeln. Bei den Ultraschallwandlern kann es sich hierbei insbesondere um Ultraschallwandler handeln, welche auf der Basis von piezoelektrischen Bauelementen arbeiten. Alternativ oder zusätzlich sind auch kapazitive mikrostrukturierte Ultraschallwandler (engl. *capacitive micromachined ultrasonic transducers,* CMUT) möglich, welche auf der Basis von Änderungen der Kapazität einer mittels Mikrostrukturtechnik in ein Siliziumsubstrat eingebrachten Mikrokavität arbeiten.

Aus der Gruppe der Ultraschallwandler ist hierbei mindestens einer der Ultraschallwandler, vorzugsweise eine Mehrzahl von Ultraschallwandlern, als Ultraschallsender eingerichtet. Der Begriff des "Ultraschallsenders" bezeichnet hierbei einen Ultraschallwandler, welcher dazu ausgestattet ist, um während einer Sendephase, in welcher der Hochspannungsverstärker an seinem Ausgang Hochspannungssignale bereitstellt, Ultraschallsignale erzeugen, welche insbesondere dazu eingesetzt werden können, um ein zu untersuchendes Objekt zu beaufschlagen. Hierbei können die Ultraschallsender eine hohe Bandbreite und eine omnidirektionale Abstrahlcharakteristik aufweisen, wobei auf eine Fokussierung der Ultraschallsignale verzichtet werden kann. Bei dem "Objekt" kann es sich hierbei insbesondere um einen Körperteil eines Patienten oder einer Patientin handeln, bevorzugt um eine Brust einer Patientin, welche mit den so erzeugten Ultraschallsignalen direkt oder, vorzugsweise, indirekt über ein sich zwischen dem Ultraschallsender und dem Objekt befindliches Ankopplungsmedium, welches das zu untersuchende Objekt benetzt, beaufschlagt werden.

Weiterhin ist aus der Gruppe der Ultraschallwandler mindestens einer der Ultraschallwandler, vorzugsweise eine Mehrzahl von Ultraschallwandlern, als Ultraschallempfänger zur Aufnahme von Ultraschallmesssignalen eingerichtet, welche durch Transmission durch das Objekt oder eines Teils davon und/oder durch Reflexion an dem Objekt oder eines Teils davon erzeugt werden können. Obwohl die von den Ultraschallsendern emittierten Ultraschallsignale hohe Signalstärken von 10 V bis 120 V, bevorzugt von 80 V bis 120 V erreichen können, während die erfassten Ultraschall-Messsignale geringe Signalstärken von lediglich 1 nV bis 10 mV, vorzugsweise von 1 $\mu$V bis 100 $\mu$V, erreichen können, ist die vorliegende Vorrichtung derart ausgestaltet, dass damit dennoch eine Trennung der erfassten Ultraschallmesssignale von den erzeugten Ultraschallsignalen möglich ist.

Hierbei kann einerseits jeder der Ultraschallwandler gleichzeitig als Ultraschallsender und als Ultraschallempfänger eingesetzt werden. Andererseits können jedoch auch derartige Ultraschallwandler eingesetzt werden, welche entweder nur als Ultraschallsender oder nur als Ultraschallempfänger dienen können. Weiterhin kann im Vergleich zu den Ultraschallsendern eine höhere Anzahl an Ultraschallempfängern vorgesehen sein, um so eine höhere Auflösung, vorzugsweise eine höhere räumliche Auflösung, der Ultraschallsignale erzielen zu können. Um eine möglichst platzsparende Ausführung der vorliegenden Vorrichtung und eine möglichst enge Abtastung des zu untersuchenden Objekts in drei Dimensionen zu ermöglichen, kann im letzteren Fall ein Teil der Ultraschallwandler gleichzeitig sowohl als Ultraschallsender als auch als Ultraschallempfänger eingesetzt werden, während weitere Ultraschallwandler nur als Ultraschallempfänger dienen können. Weitere Ausgestaltungen sind jedoch möglich. Darüber hinaus kann es für eine Verwendung der vorliegenden Vorrichtung genügen, wenn die eingesetzten Ultraschallempfänger lediglich als weniger aufwändige, nicht-fokussierte Sensoreinrichtungen ausgestaltet sind.

In einer bevorzugten Ausgestaltung kann der erste, als Eingangskanal ausgestaltete Bereich weiterhin über einen Eingangsschalter verfügen, welcher zumindest auf einen der Werte AN oder AUS eingestellt werden kann. Auf diese Weise kann, sofern der Eingangsschalter auf "AN" gestellt ist, eine Beaufschlagung des Hochspannungsverstärkers durch die externen analogen Hochspannungssignale ermöglicht oder, sofern der Eingangsschalter alternativ auf "AUS" gestellt ist, unterbunden werden.

In einer weiteren bevorzugten Ausgestaltung kann der erste, als Eingangskanal ausgestaltete Bereich weiterhin einen einstellbaren Filter aufweisen, welcher dazu eingerichtet sein kann, um niedrige Frequenzanteile aus den vom Ultraschallempfänger empfangenen Ultraschallmesssignalen herauszufiltern. In einer besonderen Ausgestaltung kann der einstellbare Filter hierbei über einen einstellbaren elektrischen Widerstand und einen Kondensator verfügen, welche in Parallelschaltung angeordnet sind. Andere Arten von Filtern sind jedoch ebenfalls möglich, z.B. sog. Switched Capacitor Filter, GmC Filter, ADC-Filter oder Digitalfilter.

Der erste Bereich als Eingangskanal ist dazu ausgestattet, um mindestens ein Messsignal an den als Ausgangskanal eingerichteten zweiten Bereich auf dem Substrat zu übergeben. Bei dem Messsignal kann es sich hierbei um ein durch einen Ultraschallempfänger bereitgestelltes ungefiltertes Ultraschallmesssignal handeln, alternativ, oder um ein entsprechend gefiltertes Signal, insbesondere um ein Ausgangssignal des beschriebenen einstellbaren Filters. Die Übergabe des Messsignals aus dem Eingangskanal in den Ausgangskanal kann grundsätzlich mittels einer einfachen elektrisch leitenden Verbindung zwischen dem Eingangskanal und dem Ausgangskanal erfolgen. Erfindungsgemäß wird hierzu jedoch eine Multiplexer-Einrichtung, welche über mindestens einen Multiplexer, vorzugsweise eine Mehrzahl von Multiplexern, verfügen kann, verwendet. Bei dem "Multiplexer" handelt es sich hierbei um einen Schaltkreis, welcher dazu eingerichtet ist, um Ultraschallmess-signale, welche von mindestens zwei voneinander verschiedenen Ultraschallwandlern aufgenommen wurden, direkt oder nach optional erfolgter Filterung, zu einem gemeinsamen Messsignal zu vereinen, das dem Ausgangskanal übergeben werden kann. In einer besonders bevorzugten Ausgestaltung kann der mindestens eine Multiplexer als 3:1 Multiplexer ausgestaltet sein, welcher drei gesonderte Messsignale zu einem gemeinsamen Messsignal vereinen kann. Andere Arten von Multiplexern, insbesondere ein 2:1 Multiplexer, der zwei Messsignale zu einem gemeinsamen Messsignal vereinen kann, oder ein 4:1 Multiplexer, der vier Messsignale zu einem gemeinsamen Messsignal vereinen kann, sind ebenfalls möglich. Durch einen Einsatz von Multiplexern kann insbesondere die Anzahl von für eine Signalauswertung erforderlichen Signalkanälen verringert werden.

Der zweite, als Ausgangskanal ausgestaltete Bereich umfasst mindestens einen rauscharmen Niederspannungsvorverstärker, mindestens einen Ausgangsschalter und mindestens einen Niederspannungssignalausgang. Der mindestens eine rauscharme Niederspannungsvorverstärker ist hierbei für einen Empfang des von dem zugehörigen Multiplexer bereitgestellten Messsignals ausgestattet. Der Niederspannungsvorverstärker weist hierbei CMOS-Halbleiterbauelemente, insbesondere CMOS-Transistoren, auf, welche, im Unterschied zu den Hochvolt-CMOS-Halbleiterbauelementen in dem Hochspannungsverstärker, für Empfang, Weiterverarbeitung und Ausgabe von Niederspannung eingerichtet sind. Insbesondere eignet sich hierfür eine Standard-Verstärkerschaltung, welche eine gefaltete Kaskade und einen Eingangstransistor aufweisen kann. Um die Verstärkung zu erhöhen, können zwei derartige Verstärker in Serie geschaltet werden. Der Verstärker befindet sich vorzugsweise in einer n-Wanne.

Was Bandbreite der in der vorliegenden Vorrichtung eingesetzten Verstärker und Linearität der zugehörigen Verstärkung betrifft, so können der sich im Eingangskanal befindliche Hochspannungsverstärker eine erste Bandbreite und eine erste Linearität sowie der im Ausgangskanal angeordnete Niederspannungsvorverstärker eine zweite Bandbreite und eine zweite Linearität aufweisen, wobei sich in einer besonders bevorzugten Ausgestaltung, innerhalb einer üblichen Toleranz, die erste Bandbreite und die zweite Bandbreite und/oder die erste Linearität und die zweite Linearität einander entsprechen können. Diese besonders bevorzugte Ausgestaltung kann damit eine gute Rauschanpassung und hohe Signaltreue für eine Wiedererkennung der Ultraschallmesssignale ermöglichen, insbesondere im Hinblick auf Nachweis und Trennung von Reflexionssignalen von Transmissionssignalen.

Der Ausgangsschalter in dem zweiten Bereich kann zumindest auf einen der Werte AN oder AUS eingestellt werden kann. Auf diese Weise kann zumindest während der Sendephase des mindestens einen Ultraschallsenders, sofern der Ausgangsschalter auf "AN" gestellt ist, eine Beaufschlagung des Niederspannungsvorverstärkers mit dem Messsignal ermöglicht oder, sofern der Ausgangsschalter alternativ auf "AUS" gestellt ist, unterbunden werden. Vorteilhaft an dieser Funktion des Ausgangsschalters ist, dass auf diese Weise während der Sendephase des mindestens einen Ultraschallsenders verhindert werden kann, dass Hochspannungssignale, welche für den Betrieb des Ultraschallsenders verwendet werden, den Niederspannungsvorverstärker beaufschlagen und somit möglicherweise beschädigen oder zerstören können.

Der ebenfalls im Ausgangskanal angeordnete Niederspannungssignalausgang ist zu einer Übertragung eines mit dem Niederspannungsvorverstärker vorverstärkten Messsignals an eine externe Steuer- und Auswerteeinheit, welche Bestandteil eines mit der vorliegenden Vorrichtung ausgestatteten Ultraschall-Computertomographen sein kann, eingerichtet. Die externe Steuer- und Auswerteeinheit kann darüber auch zur Bereitstellung von analogen Hochspannungssignalen an den mindestens einen, sich in dem Eingangskanal befindlichen Hochspannungssignaleingang eingesetzt werden.

In einem weiteren Aspekt betrifft die vorliegende Erfindung einen Ultraschall-Computertomographen. Der Ultraschall-Computertomograph umfasst hierbei

- einen Behälter, welcher zur Aufnahme eines zu untersuchenden Objekts und eines Ankopplungsmediums eingerichtet ist;
- mindestens eine Vorrichtung zur Ansteuerung und Auslese einer Gruppe von Ultraschallwandlern, welche an mindestens einer Wandung des Behälters angebracht sind; und
- eine Steuer- und Auswerteeinheit zur Bereitstellung von analogen Hochspannungssignalen für mindestens einen Hochspannungssignaleingang der Vorrichtung sowie zur Aufnahme und Auswertung von Ultraschallmesssignalen, welche durch mindestens einen Niederspannungssignalausgang der Vorrichtung bereitgestellt werden.

Wie erwähnt, weist der Ultraschall-Computertomograph einen, vorzugsweise in eine Patientenliege eingebrachten Behälter aus, welcher an mindestens einer Wandung über eine Gruppe von Ultraschallwandlern verfügt und welcher dazu eingerichtet ist, um das zu untersuchende Objekts und ein das Objekt benetzende Ankopplungsmedium aufzunehmen. Auf diese Weise kann erreicht werden, dass eine Ankopplung und Übertragung von Ultraschallsignalen zwischen den Ultraschallwandlern und dem zu untersuchenden Objekt möglichst verlustfrei ausgestaltet ist.

Insbesondere für medizinische Zwecke und zur zerstörungsfreien Prüfung kann der Ultraschall-Computertomograph über eine hohe Anzahl, vorzugsweise von mindestens 100, Ultraschallwandlerköpfe ("Ultraschallwandlerarrys") verfügen, wobei jeder der Ultraschallwandlerköpfe mehrere, vorzugsweise über 4 bis 18, Emitter und mehrere, vorzugsweise 9 bis 18, Empfänger aufweisen kann, welche über eine hohe Anzahl von Datenkanälen, vorzugsweise von 320 Datenkanälen, ausgelesen werden können. Vorzugsweise ist hierbei die Anzahl der Emitter und die Anzahl der Empfänger gleich.

Für weitere Einzelheiten in Bezug auf den Ultraschall-Computertomographen wird auf das unten stehende Ausführungsbeispiel und die Beschreibung der erfindungsgemäßen Vorrichtung verwiesen.

Die vorliegende Vorrichtung und ein damit ausgestatteter Ultraschall-Computertomograph eignen sich insbesondere für einen Einsatz in ultraschallbasierten Bildgebungsverfahren für die Brustkrebsfrüherkennung. Weitere Einsatzzwecke, insbesondere in der zerstörungsfreien Materialprüfung oder der Testtechnik, sind ebenfalls möglich.

Vorteile der Erfindung

Die vorgeschlagene Vorrichtung zur Ansteuerung und Auslese einer Gruppe von Ultraschallwandlern für die Ultraschall-Computertomographie weist im Vergleich zu aus dem Stand der Technik bekannten Vorrichtungen wesentliche Vorteile auf.

Die vorliegende Vorrichtung verfügt über eine reproduzierbare Sensorcharakteristik sowie trotz kleiner aktiver Sensorfläche über ein hohes Signal-Rausch-Verhältnis und eine hohe Signaldynamik bei einem niedrigen Gesamtpreis. Hierbei sind die Ultraschallempfänger für Signalstärken von 1 nV bis 10 mV eingerichtet, während Signalstärken der UltraschallSender für eine omnidirektionale Abstrahlcharakteristik, eine große Bandbreite sowie nicht-fokussierten Ultraschall 80 V bis 120 V erreichen können. Aufgrund der damit gegebenen hohen Signaldynamik kann der Ultraschallwandler gleichzeitig kleine Reflexionssignale und großen Transmissionssignale registrieren. Darüber weisen die Ultraschallsender und die Ultraschallempfänger weitegehend gleiche Bandbreiten und eine gute Linearität auf, womit eine gute Rauschanpassung sowie eine große Signaltreue für eine Wiedererkennung der Signale, insbesondere für den Nachweis und die Trennung von Transmissions- und Reflexionssignalen, möglich werden. Durch einen Verzicht auf eine Integration einer Fokussierungstechnik für eine räumliche Signal-Abtastung in die vorliegende Vorrichtung, welche genauso in der Rekonstruktion in dem vorgeschlagenen Ultraschall-Computertomograph durchgeführt werden kann, vereinfacht sich der Aufbau der vorliegenden Vorrichtung erheblich, wodurch auch die Datenaufnahme deutlich schneller ausgeführt werden kann.

Weiterhin können ein oder mehrere Gruppen an Ultraschallwandlern des Ultraschall-Computertomographen ein Abtasten des Raumes in drei Dimensionen ermöglichen, ohne dass hierfür eine mechanische Bewegung der Ultraschallwandler, welche jedoch dennoch möglich bleibt, erforderlich ist. Insbesondere können hierbei mindestens 100 Vorrichtungen über eine dreidimensionale Messgeometrie in Form einer "Apertur" verteilt angeordnet sein, wobei jede Vorrichtung über mindestens 3, bevorzugt mindestens 18, Ultraschallwandler verfügen kann, die als Sender und/oder als Empfänger arbeiten können und so eine schnelle Datenaufnahme ermöglichen. Dennoch bleibt eine Anpassung eines jeden einzelnen Ultraschallwandlers an unterschiedliche, in einem zu untersuchenden Objekt, insbesondere einem Körperteil, auftretende Abschwächungen möglich, insbesondere eine Anpassung an Größe, Zusammensetzung und/oder Struktur des betreffenden Objekts.

Kurze Beschreibung der Figuren

Weitere Einzelheiten und Merkmale der vorliegenden Erfindung ergeben sich aus der nachfolgenden Beschreibung von bevorzugten Ausführungsbeispielen, insbesondere in Verbindung mit den abhängigen Ansprüchen. Hierbei können die jeweiligen Merkmale für sich alleine oder zu mehreren in Kombination miteinander verwirklicht sein. Die Erfindung ist jedoch nicht auf die Ausführungsbeispiele beschränkt. Die Ausführungsbeispiele sind schematisch in den nachfolgenden Figuren dargestellt. Hierbei bezeichnen gleiche Bezugsziffern in den Figuren gleiche oder funktionsgleiche Elemente bzw. hinsichtlich ihrer Funktionen einander entsprechende Elemente.

Im Einzelnen zeigen:

Figur 1 ein Blockschaltbild eines bevorzugten Ausführungsbeispiels für eine Vorrichtung zur Ansteuerung und Auslese eines einzelnen Ultraschallwandlers;

Figur 2 ein Blockschaltbild eines bevorzugten Ausführungsbeispiels für eine Multiplexer-Einrichtung zur Ansteuerung und Auslese einer Gruppe von Ultraschallwandlern;

Figur 3 eine schematische Darstellung eines Eingangssignals über drei Verstärkerstufen (Fig. 3a bis 3c) und einer zugehörigen Signalantwort (Fig. 3d);

Figur 4 ein Schaltbild eines bevorzugten Ausführungsbeispiels für einen Hochspannungsverstärker;

Figur 5 ein Schaltbild eines bevorzugten Ausführungsbeispiels für einen Rückkopplungsschaltkreis, der den Hochspannungsverstärker aus Figur 4 umfasst; und

Figur 6 eine schematische Darstellung eines bevorzugten Ausführungsbeispiels für einen erfindungsgemäßen Ultraschall-Computertomographen.

9

Beschreibung der Ausführungsbeispiele

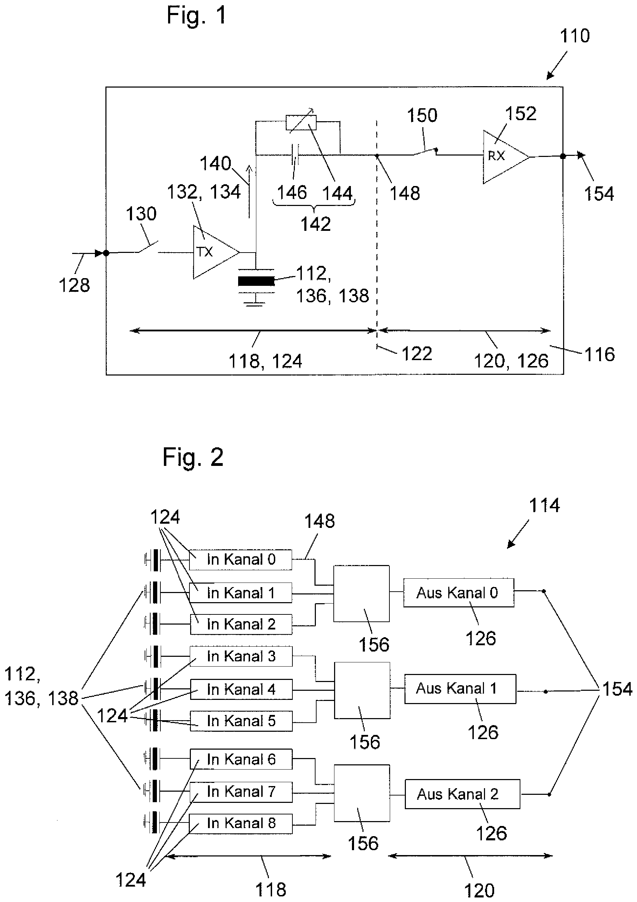

Figur 1 zeigt ein Blockschaltbild einer erfindungsgemäßen Vorrichtung 110 zur Ansteuerung und Auslese einer Gruppe von Ultraschallwandlern, wobei Figur 1 aus Gründen der Einfachheit der Darstellung lediglich einen einzelnen Ultraschallwandler 112 zeigt. Ein bevorzugtes Ausführungsbeispiel für die Ansteuerung und Auslese einer Mehrzahl von Ultraschallwandlern 112 ist in Figur 2 dargestellt, welche ein Blockschaltbild für eine für diesen Zweck eingerichtete Multiplexer-Einrichtung 114 zeigt.

Die in Figur 1 dargestellte Vorrichtung 110 zur Ansteuerung und Auslese einer Gruppe von Ultraschallwandlern 112 verfügt über ein gemeinsames Substrat 116, welches zur Aufnahme von elektronischen Bauelementen eingerichtet ist. Das gemeinsame Substrat 116 weist einen ersten Bereich 118 und einen elektrisch hiervon abgetrennten zweiten Bereich 120 auf, wobei eine Abtrennung 122 des ersten Bereichs 118 von dem zweiten Bereich 120 mittels n-Wannen (nicht dargestellt) erfolgt. Die Abtrennung 122 der beiden Bereiche 118, 120 durch n-Wannen kann es insbesondere ermöglichen, Niederspannungs-Bauelemente auf dem zweiten Bereich 120 vor der auf dem ersten Bereich 118 auftretenden Hochspannung zu schützen und eine Ausbreitung von mögliche Störungen von dem ersten Bereich 118 auf den zweiten Bereich 120 möglichst weitgehend zu unterdrücken. Während der erste Bereich 118 als Eingangskanal 124 dazu ausgelegt ist, um durch externe, analoge Hochspannungssignale ansteuerbare Hochspannungselemente aufzunehmen, ist der zweite Bereich 120 als Ausgangskanal 126 zur Aufnahme von Niederspanungselementen eingerichtet, welche dazu ausgelegt sind, um ein Ultraschall-Messsignale vorzuverstärken und an eine externe Steuer- und Auswerteeinheit weiterzuleiten.

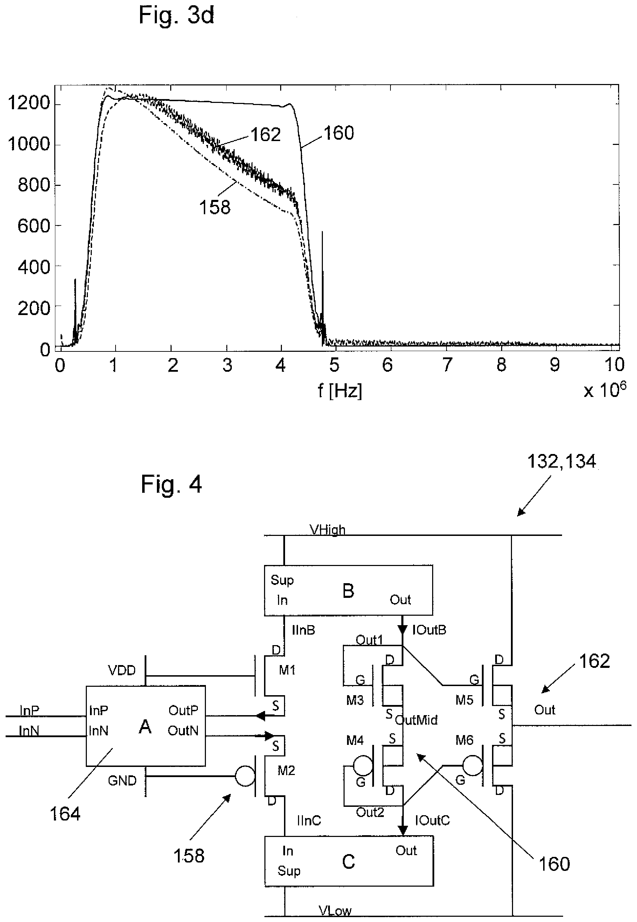

Der zur Aufnahme der Hochspannungselemente ausgelegte Eingangskanal 124 umfasst einen Hochspannungssignaleingang 128, welcher dazu eingerichtet ist, um analoge Hochspannungssignale, welche von einem externen Hochspannungssender (nicht dargestellt) bereitgestellt werden, aufzunehmen und über einen Eingangsschalter 130 und einen Hochspannungsverstärker 132 an den Ultraschallwandler 112 zu führen. Der Eingangsschalter 130 kann hierbei auf AN oder auf AUS eingestellt werden, um eine Beaufschlagung des Hochspannungsverstärker 132 durch die externen analogen Hochspannungssignale zu ermöglichen (Eingangsschalter 130 AN) oder zu unterbinden (Eingangsschalter 130 AUS). Der in Figur 1 schematisch dargestellte Hochspannungsverstärker 132 verfügt über elektronische Bauelemente, welche in Form von Hochvolt-CMOS-Halbleiterbauelementen ausgestaltet sind. Hierbei weist der Hochspannungsverstärker 132 mindestens einen analogen, linear rückgekoppelten Verstärker 134 auf, welcher in Figur 4 im Einzelnen dargestellt ist. Der Hochspannungsverstärker 132 ermöglicht auf einfache Weise eine Anpassung der Ansteuerung jedes einzelnen Ultraschallwandlers 112 an unterschiedliche in einem zu untersuchenden Objekt, insbesondere einem Körperteil, auftretende Abschwächungen, vorzugsweise eine Anpassung an Größe, Zusammensetzung und/oder Struktur des Objekts.

Eine besonders bevorzugte Ausführungsform des Hochspannungsverstärkers 132 findet sich in Figur 4.

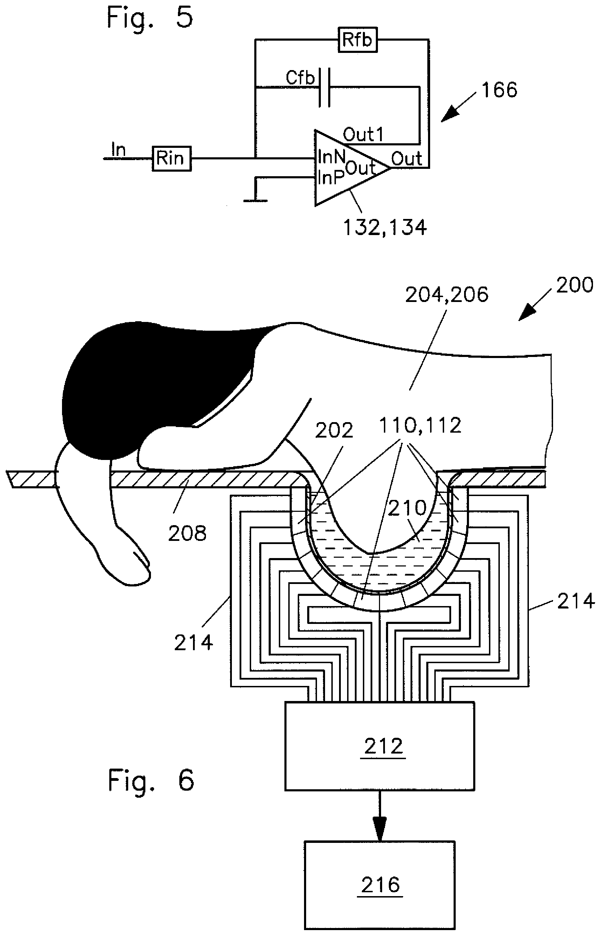

Der Eingangskanal 124 der Vorrichtung 110 umfasst weiterhin die oben erwähnte Gruppe von Ultraschallwandlern 112, von denen in Figur 1 lediglich ein einzelner Ultraschallwandler 112 schematisch dargestellt ist. Der Ultraschallwandler 112 dient hierbei einerseits als Ultraschallsender 136 und ist in dieser Eigenschaft dazu eingerichtet ist, um während einer Sendephase, in welcher sowohl der Eingangsschalter 130 auf AN eingestellt ist als auch der Hochspannungsverstärker 132 von einer Intensität = Null verschiedene Hochspannungssignale an seinem Ausgang bereitstellt, Ultraschallsignale zu erzeugen. Der Ultraschallwandler 112 kann insbesondere als piezoelektrisches Bauelement ausgeführt sein. Hierbei kann das piezoelektrische Bauelement ein vorzugsweise in Form eines dünnen Plättchens vorliegendes Komposit aus Blei, Zirkonat und Titanat, üblicherweise als "PZT" abgekürzt, aufweisen, wobei das Plättchen auf den ersten Bereich 118 des Substrats aufgebracht, insbesondere aufgeklebt, werden und eine Kontaktierung mittels Drahtbonden (*wire bonding*) erfolgen kann. Andere Arten der Ausführung des Ultraschallsenders 136 sind jedoch möglich. Die mittels des Ultraschallsenders 136 erzeugten Ultraschallsignale werden insbesondere dazu eingesetzt, um das zu untersuchende Objekt, insbesondere das Körperteil, oder, bevorzugt, ein Ankopplungsmedium, welches das zu untersuchende Objekt benetzt, zur Ankopp1ung und Übertragung der Ultraschallsignale zwischen dem Ultraschallwandler 112 und dem zu untersuchenden Objekt zu beaufschlagen. Für weitere Einzelheiten wird auf die Darstellung in Figur 6 verwiesen.

Der in Figur 1 schematisch dargestellte Ultraschallwandler 112 dient im vorliegenden Ausführungsbeispiel gleichzeitig auch als Ultraschallempfänger 138 zur Aufnahme von Ultraschallsignalen. Im Allgemeinen können jedoch auch Ultraschallwandler eingesetzt werden, welche entweder nur als Ultraschallsender oder nur als Ultraschallempfänger eingesetzt werden können. Hierbei kann insbesondere eine höhere Anzahl an Ultraschallempfängern vorgesehen werden, um eine höhere Auflösung, vorzugsweise eine höhere räumliche Auflösung, der Ultraschallsignale erzielen zu können. Zur möglichst platzsparenden Ausführung der Vorrichtung 110 kann im letzteren Fall ein Teil der Ultraschallwandler gleichzeitig sowohl als Ultraschallsender als auch als Ultraschallempfänger dienen, während weitere Ultraschallwandler nur als Ultraschallempfänger eingesetzt werden können. Weitere Ausführungen sind jedoch möglich.

Aus dem vom Ultraschallempfänger 138 empfangenen Ultraschallmesssignale 140 können hierbei zunächst mittels eines einstellbaren Filters 142, welcher einen einstellbaren Widerstand 144 und einen Kondensator 146 in Parallelschaltung aufweisen kann, niedrige Frequenzanteile aus den Ultraschallmesssignalen 140 herausgefiltert werden und als Messsignal 148 aus dem Eingangskanal 124 an den Ausgangskanal 126 der Vorrichtung 110 übergeben werden. Hierbei lassen sich insbesondere Frequenzanteile unterhalb einer Grenzfrequenz aus den Ultraschallmesssignalen 140 herausfiltern, wobei die "Grenzfrequenz" eine einstellbare Frequenz angibt, unterhalb welcher der Filter 142 mindestens einen vorgegebenen Wert, beispielsweise die Hälfte (50 %) oder $1/e$ ($\approx$ 36,8 %), der zugehörigen Ultraschallmesssignale 140 herausfiltern kann. Eine Übergabe des Messsignals 148 kann, wie in Figur 1 schematisch dargestellt, mittels einer einfachen elektrisch leitenden Verbindung erfolgen. Erfindungsgemäß wird hierzu im Allgemeinen jedoch die in Figur 2 schematisch dargestellte Multiplexer-Einrichtung 114 eingesetzt.

Der zur Aufnahme der Niederspannungselemente ausgelegte Ausgangskanal 126 umfasst einen Ausgangsschalter 150, mindestens einen Niederspannungsvorverstärker 152 und einen Niederspannungssignalausgang 154. Der Ausgangsschalter 150 kann hierbei auf AN oder auf AUS eingestellt werden, um eine Beaufschlagung des Niederspannungsvorverstärkers 152 durch das Messsignals 148 zu ermöglichen (Ausgangsschalter 150 AN) oder zu unterbinden (Ausgangsschalter 150 AUS). Damit ist der Ausgangsschalter 150 dazu eingerichtet ist, um zumindest während der Sendephase des Ultraschallsenders 136 eine Beaufschlagung des Niederspannungsvorverstärkers 152 zu einer weiteren Bearbeitung des von dem Eingangskanal 124 bereitgestellten Messsignals 148 zu verhindern.

Der Niederspannungsvorverstärker 152 umfasst hierbei mindestens einen rauscharmen Vorverstärker, welche für einen Signalempfang von Ultraschallsignalen eingerichtet ist, wobei die hierin verstärkten Niederspannungssignale dem Niederspannungssignalausgang 154 zugeführt werden, wobei der Niederspannungssignalausgang 154 zu einer Kommunikation mit einer externen Steuer- und Auswerteeinheit eingerichtet ist. Hierbei können bevorzugt die verstärkten Niederspannungssignale an die externe Steuer- und Auswerteeinheit weitergeleitet werden, etwa um eine Rekonstruktion von Echtzeit-Abbildungen auf einem Bildschirm während einer Messung an dem Objekt, insbesondere dem Körperteil, vornehmen zu können.

Der Niederspannungsvorverstärker 152 verfügt über CMOS-Halbleiterbauelemente, insbesondere Transistoren, die als herkömmliche CMOS-Transistoren für den Einsatz bei Niederspannung eingerichtet sind. Der im vorliegenden Ausführungsbeispiel eingesetzte Niederspannungsvorverstärker 152 verfügt hierzu über eine Standard-Verstärkerschaltung, welche eine gefaltete Kaskade und einen Eingangstransistor aufweist, wobei sich der Verstärker vorzugsweise in einer n-Wanne befindet. Um die Verstärkung zu erhöhen, können zwei derartige Verstärker in Serie geschaltet werden (nicht dargestellt).

Wie bereits erwähnt, zeigt Figur 2 ein Blockschaltbild für eine Multiplexer-Einrichtung 114, welche in der beispielhaft dargestellten Ausführung dazu eingerichtet ist, um jeweils drei Messsignale 148 von mindestens drei, jeweils auf dem ersten Bereich 118 des Substrats 116 angeordneten Eingangskanälen 124 mittels eines 3:1 Multiplexers 156 zu einem gemeinsamen Messsignal zu vereinen. Andere Arten von Multiplexern 156, beispielsweise ein 2:1 Multiplexer, welcher zwei Messsignale 148 zu einem gemeinsamen Messsignal vereinen kann, oder ein 4:1 Multiplexer, welcher vier Messsignale 148 zu einem gemeinsamen Messsignal vereinen kann, sind ebenfalls möglich. Der innere Aufbau eines Multiplexers 156 ist dem Fachmann, z. B. aus D.F. Lemmerhirt, s.o., bekannt.

Zur Herstellung einer erfindungsgemäßen Vorrichtung kann vorzugsweise eine spezielle Hochvolt-CMOS-Technologie des Unternehmens AMS eingesetzt werden, welche es erlaubt, gleichzeitig standardisierte Niedervolt-CMOS Transistoren (35 $\mu$m oder 18 $\mu$m) und Hochvolt-CMOS-Transistoren auf einem einzigen Substrat (Chip) bereitzustellen, wobei sich eine Spannungsfestigkeit bis mindestens 100 V, vorzugsweise bis zu 120 V, erzielen lässt. Im Gegensatz hierzu wäre ein Einsatz einer reinen Hochspannungs-Technologie nachteilhaft, da sich damit rauscharme Vorverstärker mit einem Signal-Rausch-Verhältnis von mindestens 60 dB und mit Bandbreiten von mindestens 1MHz nicht erzielen lassen. Eine hier vorgeschlagene Kombination von Hochspannungs- und Niederspannungs-Technologien auf einem gemeinsamen Substrat ermöglicht vielmehr den Aufbau eines Ultraschall-Computertomographen für eine dreidimensionale Messwerterfassung unter den hierfür gegebenen spezifischen Anforderungen.

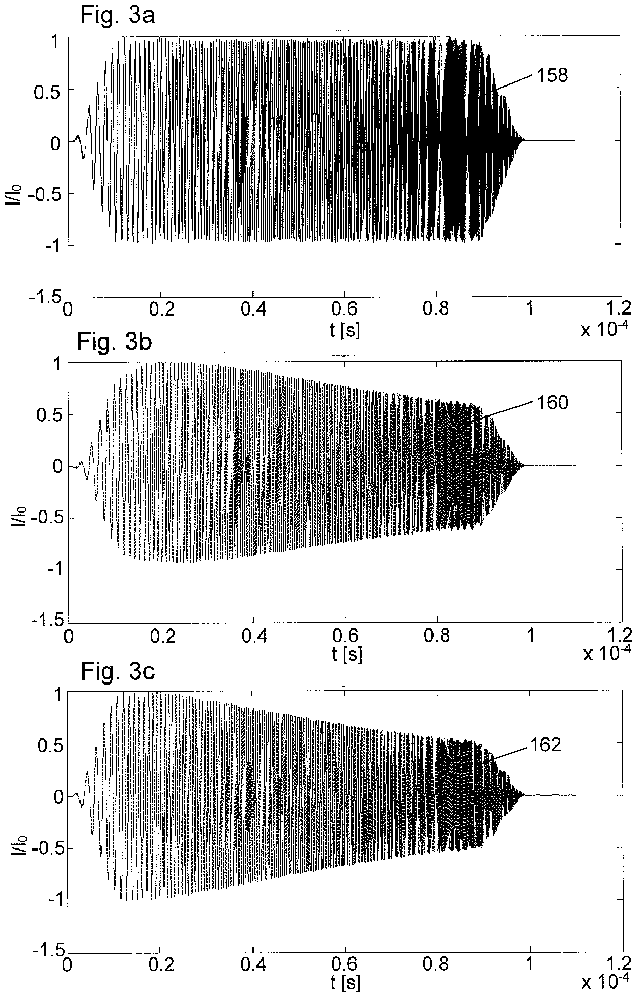

Die Figuren 3a bis 3c zeigen eine normalisierte Signalamplitude $I/I_0$ eines breitbandigen Eingangssignals, welches über die drei aufeinanderfolgenden Verstärkerstufen 158, 160, 162 des Hochspannungsverstärkers 132 im Zeitbereich t [s] darstellt ist. Figur 3d zeigt eine jeweilige Signalantwort des Hochspannungsverstärkers 132 über die drei Verstärkerstufen 158, 160, 162 in Form als Signalamplitude I im Frequenzbereich f [Hz]. Hieraus ergibt sich, dass das breitbandige Signal über seine gesamte Bandbreite erhalten bleibt; abgesehen von einer geringfügigen Dämpfung der Amplitude bei höheren Frequenzen, treten keine signifikanten Phasensprünge oder Phasenverschiebungen zwischen den Frequenzen auf; diese Eigenschaft kann in vorteilhafter Weise insbesondere eine spätere Signalverarbeitung u.a. mit einem sog. *Matched Filter* Verfahren ermöglichen.

Figur 4 zeigt ein schematisches Schaltbild eines bevorzugten Ausführungsbeispiels für den analogen, linearen, rückgekoppelten Verstärker 134, welcher als in der Ansteuerung des Ultraschallwandlers 112 eingesetzt wird. Der analoge, lineare, rückgekoppelte Verstärker 134 umfasst hierbei zumindest drei Blöcke A, B, C, welche wie im Folgenden im Einzelnen beschrieben ausgestaltet sind.

Der Block A umfasst einen Differentialverstärker 164, welcher zwei Eingänge InP und InN sowie zwei Ausgänge OutP und OutN aufweist. Hierbei erzeugen die beiden Ausgänge OutP und OutN jeweils einen Ausgangsstrom IOutP bzw.

IOutN, wobei eine Differenz von Absolutwerten der Ausgangsströme |IOutP-IOutN| gemäß Gleichung (1) proportional zur Differenz der Eingangsspannungen UInP bzw. UInN an den beiden Eingängen InP und InN ist:

$$|IOutP\text{-}IOutN| \sim |UInP\text{-}UInN|. \tag{1}$$

In Bezug auf die Richtung der Ausgangsströme IOutP bzw. IOutN ist anzumerken, dass der Ausgangsstrom IOutP am Ausgang OutP in den Block A hineinfließt, während der Ausgangsstrom IOutN am Ausgang OutN aus dem Block A herausfließt.

Der Ausgang OutP des Differentialverstärkers 164 in Block A wird mit einem ersten Transistor M1 verbunden, während der Ausgang OutN des Differentialverstärkers in Block A mit einem zweiten Transistor M2 verbunden wird. Wie in Figur 4 dargestellt, ist der erste Transistor M1 als n-Kanal MOSFET (NMOS) ausgeführt, während der zweite Transistor M2 als p-Kanal MOSFET (PMOS) ausgeführt ist. Hierbei verfügt jeder der Transistoren M1 und M2 über die Ausgänge Source S, Drain D und Gate G. Hierbei ist das Gate G des ersten Transistors M1 mit einer Niederspannung VDD verbunden, während das Gate G des zweiten Transistors M2 mit einer Niederspannungserde GRD verbunden ist, wobei die Niederspannung VDD und die Niederspannungserde GRD gleichzeitig auch zur Spannungsversorgung des Differentialverstärkers in Block A dienen. Eine typische Spannung für die Niederspannung VDD kann 3,3 V betragen, während die Niederspannungserde auf 0 V gelegt ist. Andere Werte für die Niederspannung VDD von 1 V bis 5 V sind ebenfalls möglich.

Die in Figur 4 dargestellte Ausführung hat insbesondere zur Folge, dass der erste als NMOS vorliegende Transistor M1 dazu eingerichtet ist, um eine Hochspannung mit positivem Vorzeichen zwischen dem Drain D und seinen anderen Ausgängen aufrecht zu erhalten, wobei die Hochspannung Werte von bis zu 60 V annehmen kann. In analoger Weise ist der zweite als PMOS vorliegende Transistor M2 dazu eingerichtet ist, um eine Hochspannung mit negativem Vorzeichen zwischen dem Drain D und seinen anderen Ausgängen aufrecht zu erhalten, wobei die Hochspannung Werte von bis zu -60 V annehmen kann. Diese Art von Drain D kann in beiden Fällen jeweils auch als "Hochspannungsdrain" bezeichnet werden.

Der Drain D des ersten Transistors M1 wird mit dem weiteren Block B verbunden. Hierbei weist der Block B einen Eingang In, einen Ausgang Out und einen Anschluss Sup für eine Spannungsversorgung auf. Ein aus dem Eingang In herausfließender Strom IInB entspricht hierbei ungefähr einem aus dem Ausgang Out herausfließenden Strom IOutB. Aufgrund dieser Eigenschaft kann der Block B auch als "Stromspiegel" bezeichnet werden. Der Anschluss Sup für die Spannungsversorgung ist hierbei mit einer Stromschiene VHigh verbunden. Ein typisches Potential kann hierbei 60 V betragen. Der Block B ist dazu eingerichtet, um eine Hochspannung zwischen dem Anschluss Sup für die Spannungsversorgung und dem Ausgang Out aufrecht zu erhalten. Damit kann an dem Block B eine Spannung von bis zu 120 V anliegen, ohne dass dieser beschädigt wird. Wie bereits erwähnt, kann der aus dem Eingang In herausfließende Strom IInB hierbei ungefähr dem aus dem Ausgang Out herausfließenden Strom IOutB entsprechen, sofern das zwischen dem Anschluss Sup für die Spannungsversorgung und dem Ausgang Out anliegende Potential einen Wert von ca. 500 mV überschreitet, während für geringere Potentiale unterhalb dieses Wertes der aus dem Ausgang Out herausfließende Strom IOutB niedriger ist als der aus dem Eingang In herausfließende Strom IInB. Im letzteren Fall wird daher von einer "Sättigung" des Blocks B gesprochen.

Der Drain D des zweiten Transistors M2 wird mit einem Block C verbunden. Auch der weitere Block C ist als Stromspiegel ausgestaltet. Aufgrund dieser Eigenschaft entspricht ein in den Eingang In hineinfließender Strom IInC ungefähr einem in den Ausgang Out hineinfließenden Strom IOutC. Der Anschluss Sup für die Spannungsversorgung ist hierbei mit einer Stromschiene VLow verbunden. Ein typisches Potential kann hierbei -60 V betragen. Der Block C ist dazu eingerichtet, um eine Hochspannung zwischen dem Anschluss Sup für die Spannungsversorgung und dem Ausgang Out aufrecht zu erhalten. Damit kann auch an dem Block C eine Spannung von bis zu 120 V anliegen, ohne dass dieser beschädigt wird. Auch hier kann der in den Eingang In hineinfließende Strom IInC ungefähr dem in den Ausgang Out hineinfließenden Strom IOutC entsprechen, sofern das zwischen dem Anschluss Sup für die Spannungsversorgung und dem Ausgang Out anliegende Potential einen Wert von ca. 500 mV überschreitet, während für geringere Potentiale unterhalb dieses Wertes Block C sättigt, so dass der in den Ausgang Out hineinfließende Strom IOutC niedriger ist als der in den Eingang In hineinfließende Strom IInC.

Ausgang Out1 des Blocks B ist an einen dritten Transistor M3 angeschlossen, während Ausgang Out2 des Blocks C an einen vierten Transistor M4 angeschlossen ist. Hierbei ist der dritte Transistor M3 als n-Kanal MOSFET (NMOS) ausgeführt, während der vierte Transistor M4 als p-Kanal MOSFET (PMOS) ausgeführt ist. Auch hierbei verfügt jeder der Transistoren M3 und M4 über die Ausgänge Source S, Drain D und Gate G, welche entsprechend der Figur 4 verschaltet sind. Die Transistoren M3 und M4 sind jeweils an ihren Source-Ausgängen S in Reihe geschaltet.

Die beschriebene und in Figur 4 dargestellte Verschaltung der Blöcke B und C sowie der Transistoren M3 und M4 hat zur Folge, dass die Gleichgewichts-Gleichströme IOutB und IOutC gleich groß sind. Andererseits entsprechend die Ströme IInB und IInC den an den Ausgängen OutN und OutP des Blocks A anliegenden Ausgangsströmen IPOutN und IOutP, wobei die Differenz der Ausgangsströme IPOutN und IOutP proportional zur Spannungsdifferenz |UInP-UInN| an den Eingängen InN und InP des Blocks A ist. Folglich wird bei einer niedrigen, an dem Block A anliegenden Spannungsdifferenz |UInP-UInN| entweder Block B oder Block C sättigen, während bei einer höheren an dem Block A anliegenden Spannungsdifferenz |UInP-UInN| in Bezug hierzu jeweils eine Hochspannungsverstärkung an den Ausgängen Outl des Blocks B und Out2 des Blocks C vorliegt.

Weiterhin ist der Ausgang Outl des Blocks B mit dem Gate G des Transistors M5 verbunden, während der Ausgang Out2 des Blocks C mit dem Gate G des Transistors M6 verbunden ist. Hierbei ist der Transistor M5 vom selben Typ wie der Transistor M3. Eine Breite des Transistors M5 entspricht der Breite des Transistors M3 oder die Breite des Transistors M5 ist um einen Faktor m1 größer als die Breite des Transistors M3, während die Längen der Transistoren M3 und M5 gleich sind. In Analogie ist der Transistor M6 vom selben Typ wie der Transistor M4, wobei die Breite des Transistors M6 der Breite des Transistors M4 entspricht oder um einen Faktor m2 größer ist, während die Längen der beiden Transistoren M4 und M6 ebenfalls gleich sind. Darüber hinaus sind die beiden Faktoren m1 = m2 = m gleich.

Die Transistoren M5 und M6 verfügen ebenfalls über Hochspannungsdrains, so dass sie jeweils eine Hochspannung zwischen dem Drain D und den anderen Ausgängen aufrechterhalten können. Hierbei kann das am Drain D des Transistors M5 anliegende Potential einen Wert von bis zu +120 V gegenüber dem Potential von anderen Transistorausgängen annehmen, während das am Drain D des Transistors M6 anliegende Potential einen Wert von bis zu -120 V gegenüber dem Potential von anderen Transistorausgängen annehmen ohne dass jeweils der betreffende Transistor beschädigt wird.

Ein Teilschaltkreis des in Figur 4 dargestellten Schaltkreises, wobei der Teilschaltkreis die Transistoren M3, M4, M5 und M6 umfasst, kann in derjenigen Weise eingesetzt werden, dass der durch die Transistoren M5 und M6 fließende Strom dem durch die Transistoren M3 und M4 fließenden Strom entspricht, multipliziert mit einem Faktor m. Sofern keine Last an dem Ausgang Out anliegt, entspricht gemäß Gleichung (2) das Potential UOut am Ausgang Out ungefähr dem Potential UOutMid am Ausgang OutMid in der Verbindung zwischen den Transistoren M3 und M4. Damit gilt für das an dem Ausgang Out anliegende Potential, dass

$$UOut = UOutMid + \tfrac{1}{2}(UOut1 + UOut2). \qquad (2)$$

Folglich besteht eine Hochspannungsverstärkung zwischen dem Ausgang Out in Bezug auf die an dem Block A anliegende Spannungsdifferenz |UInP-UInN|. Hierbei stellen die Transistoren M5 und M6 sicher, dass an den Ausgang Out ein niedriger Widerstand oder eine hohe Kapazität angeschlossen werden können, ohne die Hochspannungsverstärkung in der Schaltung gemäß Figur 4 signifikant zu verringern. Diese Eigenschaft beruht auf der Verbindung der Sources S der Transistoren M5 und M6. In einen Fall, in welchem das an dem Ausgang Out anliegende Potential UOut einem Anstieg des an dem Ausgang Outl anliegenden Potential UOut1 folgen sollte, wird sich die Spannung zwischen Gate G und Source S des Transistors M5 derart erhöhen, dass der Transistor M5 einen höheren Strom erzeugen wird. Dieser erhöhte Strom wird üblicherweise eine Änderung des Potentials UOut an dem Ausgang Out bewirken und somit dem ursprünglichen Anstieg entgegenwirken.

Der in Figur 4 schematisch dargestellte Schaltkreis eignet sich vorzugsweise für den Einsatz als Hochspannungsverstärker 132, insbesondere zur Ansteuerung eines piezoelektrischen Ultraschallwandlers 112. Wie Figur 5 zeigt, kann hierzu der Spannungsverstärker mit einem negativen Rückkopplungsschaltkreis 166 ausgestattet werden. Hierbei entspricht eine Signalamplitude an dem Ausgang Out des Hochspannungsverstärkers 132 der Signalamplitude an dem Eingang InN des Spannungsverstärkers, multipliziert mit einem Verhältnis Rfb/Rfln der Widerstände Rfb bzw. Rfln. Eine Kapazität Cfb kann hierbei dazu dienen, um eine Stabilität des negativen Rückkopplungsschaltkreises 166 zu erhöhen. Hierzu kann es besonders vorteilhaft sein, wie in Figur 5 dargestellt, die Kapazität Cfb mit dem Ausgang Outl des Spannungsverstärkers zu verbinden.

Figur 6 zeigt schematisch einen aus WO 2002/030288 A1 bekannten Ultraschall-Computertomographen 200, der insbesondere in einem ultraschallbasierten Bildgebungsverfahren für Brustkrebsfrüherkennung einsetzbar ist. Der Ultraschall-Computertomograph 200 umfasst einen nach oben offenen Behälter 202, in welchen ein zu untersuchender Körperteil, hier eine Brust 204 einer Patientin 206, welche auf sich einer Patientenliege 208 befindet, eingeführt wird. Im Unterschied zu der Darstellung in WO 2002/ 030288 A1, ist hier der Behälter 202 vorzugsweise in Form einer Teilsphäre ausgestaltet, um eine bessere räumliche Anpassung an die Brust 204 als das zu untersuchende Körperteil zu ermöglichen.

Der in Figur 6 schematisch dargestellte Ultraschall-Computertomograph 200 umfasst weiterhin eine Gruppe von Ultraschallwandlern 112, welche jeweils an einer Wandung des Behälters 202, möglichst gleichmäßig über eine gesamte Fläche der Wandung verteilt, fest angeordnet sind und welche jeweils eine Hauptabstrahlrichtung aufweisen, die im Wesentlichen senkrecht von der Fläche der Wandung in ein Inneres des Behälters 202 ausgerichtet ist. In den Behälter 202 kann ein Ankopplungsmedium 210 eingefüllt sein, welches den zu untersuchenden Körperteil benetzen und insbesondere zu einer besseren Ankopplung und Übertragung von Ultraschallsignalen zwischen den Ultraschallwandlern 112 und dem zu untersuchenden Körperteil dienen kann. Eine Ansteuerung und Auslese der Gruppe der Ultraschallwandler 112 erfolgt hierbei insbesondere mit der Vorrichtung 110 gemäß den Figuren 1 und 2, wobei der Hochspannungsverstärker 132 vorzugsweise entsprechend den Figuren 4 und 5 ausgestaltet sein kann.

Der hier dargestellte Ultraschall-Computertomograph 200 umfasst weiterhin eine rechnergestützte Steuer- und Auswerteeinheit 212, welche bevorzugt über einen Arbeitsspeicher verfügen und welche mit der Vorrichtung 110, bevorzugt mittels Koaxialleitungen 214, verschaltet sein kann. Andere Arten von Leitungen oder, alternativ oder zusätzlich, eine kabellose Übertragung von Daten in die Vorrichtung 110 und/oder aus der Vorrichtung 110 ist ebenfalls möglich. Die Verschaltung ist hierbei derart ausgeführt, das eine beliebige Anzahl der Ultraschallwandler 112 als Ultraschallsender 136 und/oder als Ultraschallempfänger 138 anwählbar sind, dass die von den Ultraschallsender 136 ausgesendeten Ultraschallsignale über das Ankopplungsmedium 210 den zu untersuchenden Körperteil beaufschlagen, während die von den Ultraschallempfängern 138 parallel empfangenen Ultraschallmesssignale als elektrische Signale verstärkt, gefiltert, digitalisiert und in dem Arbeitsspeicher der Steuer- und Auswerteeinheit 212 als Daten abgespeichert werden. Diese Daten können, wie insbesondere in WO 2002/030288 A1, EP 2 056 124 A1, WO 2011/124379 A2 bzw. WO 2012/110228 A1 dargestellt, ausgewertet und für eine Rekonstruktion des untersuchten Körperteils verwendet werden. Andere Auswertungs- und Rekonstruktionsverfahren können jedoch ebenfalls eingesetzt werden. Derart rekonstruierte Echtzeit-Abbildungen können in einer Ausgabeeinheit 216, insbesondere auf einem Bildschirm, während einer Messung insbesondere für medizinische Zwecke dargestellt werden. Andere Arten der Darstellung sind jedoch möglich.

Ein besonders bevorzugter Ultraschall-Computertomograph kann 157 erfindungsgemäße Vorrichtungen aufweisen, welche auch als *Transducer Array Systeme* (TAS) bezeichnet werden können, wobei jede der Vorrichtungen über 4 Ultraschallsender und über 9 Ultraschallempfänger verfügen kann. Damit kann der Ultraschall-Computertomograph insgesamt 628 Ultraschallsender und 1413 Ultraschallempfänger aufweisen, welche möglichst gleichmäßig über eine Teilsphäre verteilt sein können. Jede der Vorrichtungen kann hierbei insbesondere über eine Multiplexer-Einrichtung mit einem 3:1 Multiplexer verfügen, so dass insgesamt 471 Messsignale an den Niederspannungssignalausgängen der Vorrichtungen vorliegen, welche durch 480 dafür eingerichtete parallele Kanäle verarbeitet werden können. Darüber hinaus können durch 46 verschiedene Bewegungen der Apertur 28888 virtuelle Ultraschallsender und 64998 virtuelle Ultraschallempfänger bereitgestellt werden.

Hierbei können jeweils 2 × 2 Ultraschallwandler in Form eines Arrays mit einer Fläche von 0,9 mm × 0,9 mm eingesetzt werden, wobei jeder Ultraschallwandler über eine individuelle Fläche von 0,4 mm × 0,4 mm verfügen kann. Die maximale von den Hochspannungsverstärkern bereitgestellte Anregungsspannung kann hierbei 80 V betragen. Diese Einrichtung kann einen Öffnungswinkel für eine Abstrahlung des Ultraschalls von ca. 38° bei einer Resonanzfrequenz von ca. 2,7 MHz und einer Bandbreite von ca. 1,5 MHz ermöglichen, wodurch ein Schalldruck von ca. 6 kPa in einem Abstand von 12 cm von dem Ultraschallwandler erzielt werden kann. Andere Arten der Ausgestaltung des vorliegenden Ultraschall-Computertomographen sind jedoch möglich.

Bezugszeichenliste

| 110 | Vorrichtung |
| 112 | Ultraschallwandler |
| 114 | Multiplexer-Einrichtung |
| 116 | Substrat |
| 118 | Erster Bereich |
| 120 | Zweiter Bereich |
| 122 | Abtrennung |
| 124 | Eingangskanal |
| 126 | Ausgangskanal |
| 128 | Hochspannungssignaleingang |
| 130 | Eingangsschalter |
| 132 | Hochspannungsverstärker |
| 134 | Analoger, linearer, rückgekoppelter Verstärker |
| 136 | Ultraschallsender |
| 138 | Ultraschallempfänger |
| 140 | Ultraschallmesssignale |
| 142 | Einstellbarer Filter |
| 144 | Einstellbarer Widerstand |
| 146 | Kondensator |

148 Messsignal
150 Ausgangsschalter
152 Niederspannungsvorverstärker
154 Niederspannungssignalausgang
156 Multiplexer
158 Erste Verstärkerstufe
160 Zweite Verstärkerstufe
162 Dritte Verstärkerstufe
164 Differentialverstärker
166 Negativer Rückkopplungsschaltkreis
200 Ultraschall-Computertomograph
202 Behälter
204 Brust
206 Patientin
208 Patientenliege
210 Ankopplungsmedium
212 Steuer- und Auswerteeinheit
214 Koaxialleitung
216 Ausgabeeinheit

**Patentansprüche**

1. Vorrichtung (110) zur Ansteuerung und Auslese einer Gruppe von Ultraschallwandlern (112) für Ultraschall-Computertomographie, umfassend

   - ein gemeinsames Substrat (116), wobei das Substrat (116) einen als Eingangskanal (124) ausgestalteten ersten Bereich (118) und einen elektrisch hiervon abgetrennten, als Ausgangskanal (126) ausgestalteten zweiten Bereich (120) aufweist;
   - mindestens einen Hochspannungssignaleingang (128), welcher auf dem ersten Bereich (118) angeordnet ist, wobei der Hochspannungssignaleingang (128) dazu eingerichtet ist, um analoge Hochspannungssignale aufzunehmen;
   - mindestens einen Hochspannungsverstärker (132), welcher auf dem ersten Bereich (118) angeordnet ist, wobei der Hochspannungsverstärker (132) dazu eingerichtet ist, um die von dem Hochspannungssignaleingang (128) bereitgestellten analogen Hochspannungssignale aufzunehmen und zu verstärken, wobei der Hochspannungsverstärker (132) über Hochvolt-CMOS-Halbleiterbauelemente verfügt;
   - mindestens eine Gruppe von Ultraschallwandlern (112), welche auf dem ersten Bereich (118) angeordnet sind, wobei mindestens einer der Ultraschallwandler (112) als Ultraschallsender (136) eingerichtet ist, um während einer Sendephase, in welcher der Hochspannungsverstärker (132) an seinem Ausgang Hochspannungssignale bereitstellt, Ultraschallsignale erzeugen, und wobei mindestens einer der Ultraschallwandler (112) zur Aufnahme von Ultraschallmesssignalen (140) eingerichtet ist;
   - mindestens einen rauscharmen Niederspannungsvorverstärker (152), welcher auf dem zweiten Bereich (120) angeordnet ist, wobei der Niederspannungsvorverstärker (152) für einen Empfang des Messsignals (148) eingerichtet ist, wobei der Niederspannungsvorverstärker (152) über CMOS-Halbleiterbauelemente verfügt;
   - mindestens einen Ausgangsschalter (150), welcher auf dem zweiten Bereich (120) angeordnet ist, wobei der Ausgangsschalter (150) dazu eingerichtet ist, um zumindest während der Sendephase eine Beaufschlagung des Niederspannungsvorverstärkers (152) mit dem Messsignal (148) zu unterbinden; und
   - mindestens einen Niederspannungssignalausgang (154), welcher auf dem zweiten Bereich (120) angeordnet ist, welcher zu einer Übertragung eines vorverstärkten Messsignals (148) an eine externe Steuer- und Auswerteeinheit (212) eingerichtet ist
   **gekennzeichnet durch,**
   - mindestens einen Multiplexer (156), welcher dazu eingerichtet ist, um Ultraschallmesssignale (140), welche von mindestens zwei voneinander verschiedenen Ultraschallwandlern (112) aufgenommen wurden, zu einem gemeinsamen Messsignal (148) zu vereinen,

   wobei eine Abtrennung (122) des ersten Bereichs (118) von dem zweiten Bereich (120) auf dem Substrat (116) mittels n-Wannen erfolgt, wobei der Hochspannungsverstärker (132) mindestens einen analogen, linearen, rückgekoppelten Verstärker (134) aufweist, welcher über mindestens drei gesonderte Verstärkerstufen (158, 160, 162) verfügt, und wobei der analoge, lineare, rückgekoppelte Verstärker (134) eine Spannungsfestigkeit von mindestens 80 V aufweist.

2.  Vorrichtung (110) nach dem vorangehenden Anspruch, wobei der erste Bereich (118) für eine Aufnahme und Weiterverarbeitung von elektrischen Spannungen und Potentialen von 5 V bis 120 V ausgelegt ist und der zweite Bereich (120) für eine Aufnahme und Weiterverarbeitung elektrischen Spannungen und Potentialen von 1 nV bis 10 mV ausgelegt ist.

3.  Vorrichtung (110) nach einem der vorangehenden Ansprüche, wobei der Hochspannungsverstärker (132) eine erste Bandbreite aufweist und wobei der Niederspannungsvorverstärker (152) eine zweite Bandbreite aufweist, wobei die erste Bandbreite und die zweite Bandbreite einander entsprechen.

4.  Vorrichtung (110) nach einem der vorangehenden Ansprüche, wobei jede der Verstärkerstufen (158, 160, 162) über jeweils mindestens zwei Hochvolt-CMOS-Transistoren (M1, M2; M3, M4; M5, M6) verfügt.

5.  Vorrichtung (110) nach dem vorangehenden Anspruch, wobei sich am Eingang einer ersten Verstärkerstufe (158) ein Differentialverstärker (164) befindet, wobei die erste Verstärkerstufe (158) und eine zweite Verstärkerstufe (160) jeweils zwischen zwei Stromspiegeln (B, C) angeordnet ist, und wobei in der zweiten Verstärkerstufe (160) und in einer dritten Verstärkerstufe (162) jeweils die Hochvolt-CMOS-Transistoren (M3, M4; M5, M6) miteinander verbunden sind.

6.  Vorrichtung (110) nach einem der vorangehenden Ansprüche, wobei der erste Bereich (118) weiterhin einen Eingangsschalter (130) aufweist, welcher dazu eingerichtet ist, um eine Beaufschlagung des Hochspannungsverstärkers (132) durch die externen analogen Hochspannungssignale zu ermöglichen oder zu unterbinden.

7.  Vorrichtung (110) nach einem der vorangehenden Ansprüche, wobei der erste Bereich (118) weiterhin einen einstellbaren Filter (142) aufweist, welcher dazu eingerichtet ist, um Frequenzanteile unterhalb einer Grenzfrequenz aus den Ultraschallmesssignalen (140) herauszufiltern.

8.  Vorrichtung (110) nach einem der vorangehenden Ansprüche, wobei die Vorrichtung (110) in Form einer anwendungsspezifischen integrierten Schaltung (ASIC) vorliegt.

9.  Ultraschall-Computertomograph (200) zur Untersuchung eines Körperteils, umfassend

    - einen Behälter (202), welcher zur Aufnahme eines zu untersuchenden Objekts und eines Ankopplungsmediums (210) eingerichtet ist;
    - mindestens eine Vorrichtung (110) nach einem der vorangehenden Ansprüche zur Ansteuerung und Auslese einer Gruppe von Ultraschallwandlern (112), welche an mindestens einer Wandung des Behälters (202) angebracht sind; und
    - eine Steuer- und Auswerteeinheit (212) zur Bereitstellung von analogen Hochspannungssignalen für mindestens einen Hochspannungssignaleingang (128) der Vorrichtung (110) sowie zur Aufnahme und Auswertung von Ultraschallmesssignalen, welche durch mindestens einen Niederspannungssignalausgang (154) der Vorrichtung (110) bereitgestellt werden.

10. Ultraschall-Computertomograph nach dem vorangehenden Anspruch, ferner umfassend eine Ausgabeeinheit (216), welche zur Darstellung von rekonstruierten Echtzeit-Abbildungen eingerichtet ist.

**Claims**

1.  Device (110) for actuating and reading a group of ultrasound transducers (112) for ultrasound computer tomography, comprising

    - a common substrate (116), wherein the substrate (116) has a first region (118) configured as input channel (124) and a second region (120) configured as output channel (126), said second region (120) being separated electrically from said first region (118);
    - at least one high-voltage signal input (128) arranged on the first region (118), wherein the high-voltage signal input (128) is configured to receive analogue high-voltage signals;
    - at least one high-voltage amplifier (132) arranged on the first region (118), wherein the high-voltage amplifier (132) is configured to receive and to amplify the analogue high-voltage signals provided by the high-voltage signal input (128), wherein the high-voltage amplifier (132) has high-voltage CMOS semiconductor components;

- at least one group of ultrasound transducers (112) arranged on the first region (118), wherein at least one of the ultrasound transducers (112) is configured as ultrasound transmitter (136) to generate ultrasound signals during a transmission phase, in which the high-voltage amplifier (132) provides high-voltage signals at its output, and wherein at least one of the ultrasound transducers (112) is configured for receiving ultrasound measurement signals (140);

- at least one low-noise low-voltage preamplifier (152) arranged on the second region (120), wherein the low-voltage preamplifier (152) is configured for receiving the measurement signal (148), wherein the low-voltage preamplifier (152) has CMOS semiconductor components;

- at least one output switch (150) arranged on the second region (120), wherein the output switch (150) is configured to prevent the measurement signal (148) from being applied to the low-voltage preamplifier (152) at least during the transmission phase; and

- at least one low-voltage signal output (154) arranged on the second region (120), which is configured for transmitting a preamplified measurement signal (148) to an external control and evaluation unit (212), **characterized by**

- at least one multiplexer (156) configured to combine ultrasound measurement signals (140) that have been received from at least two mutually different ultrasound transducers (112) to form a common measurement signal (148),

wherein a separation (122) of the first region (118) from the second region (120) on the substrate (116) is effected by means of n-wells, wherein the high-voltage amplifier (132) has at least one analogue linear feedback amplifier (134) having at least three separate amplifier stages (158, 160, 162), and wherein the analogue linear feedback amplifier (134) has a dielectric strength of at least 80 V.

2. Device (110) according to the preceding claim, wherein the first region (118) is designed for reception and further processing of electrical voltages and potentials of 5 V to 120 V and the second region (120) is designed for reception and further processing of electrical voltages and potentials of 1 nV to 10 mV.

3. Device (110) according to either of the preceding claims, wherein the high-voltage amplifier (132) has a first bandwidth and wherein the low-voltage preamplifier (152) has a second bandwidth, wherein the first bandwidth and the second bandwidth correspond to one another.

4. Device (110) according to any of the preceding claims, wherein each of the amplifier stages (158, 160, 162) has in each case at least two high-voltage CMOS transistors (M1, M2; M3, M4; M5, M6).

5. Device (110) according to the preceding claim, wherein a differential amplifier (164) is situated at the input of a first amplifier stage (158), wherein the first amplifier stage (158) and a second amplifier stage (160) are arranged in each case between two current mirrors (B, C), and wherein the high-voltage CMOS transistors (M3, M4; M5, M6) are connected to one another in each case in the second amplifier stage (160) and in a third amplifier stage (162).

6. Device (110) according to any of the preceding claims, wherein the first region (118) furthermore has an input switch (130) configured to enable or to prevent the external analogue high-voltage signals from being applied to the high-voltage amplifier (132).

7. Device (110) according to any of the preceding claims, wherein the first region (118) furthermore has an adjustable filter (142) configured to filter out frequency components below a cut-off frequency from the ultrasound measurement signals (140).

8. Device (110) according to any of the preceding claims, wherein the device (110) is present in the form of an application-specific integrated circuit (ASIC).

9. Ultrasound computer tomography machine (200) for examining a body part, comprising

- a container (202) configured for receiving an object to be examined and a coupling medium (210);
- at least one device (110) according to any of the preceding claims for actuating and reading a group of ultrasound transducers (112) fitted to at least one wall of the container (202); and
- a control and evaluation unit (212) for providing analogue high-voltage signals for at least one high-voltage signal input (128) of the device (110) and also for receiving and evaluating ultrasound measurement signals provided by at least one low-voltage signal output (154) of the device (110).

**10.** Ultrasound computer tomography machine according to the preceding claim, furthermore comprising an output unit (216) configured for representing reconstructed real-time imagings.

## Revendications

**1.** Dispositif (110) pour commander et lire un groupe de transducteurs à ultrasons (112) pour réaliser une échotomographie, comprenant

- un substrat (116) commun, le substrat (116) présentant une première zone (118) conçue sous forme de canal d'entrée (124) et une deuxième zone (120), conçue sous forme de canal de sortie (126), électriquement séparée de la première zone (118) ;
- au moins une entrée de signaux haute tension (128) qui est disposée sur la première zone (118), l'entrée de signaux haute tension (128) étant conçue pour acquérir des signaux haute tension analogiques ;
- au moins un amplificateur haute tension (132) qui est disposé sur la première zone (118), l'amplificateur haute tension (132) étant conçu pour recevoir et amplifier les signaux haute tension analogiques fournis par l'entrée de signaux haute tension (128), l'amplificateur haute tension (132) comportant des composants à semi-conducteur CMOS haute tension ;
- au moins un groupe de transducteurs à ultrasons (112) qui sont disposés sur la première zone (118), au moins l'un des transducteurs à ultrasons (112) étant conçu, sous forme d'émetteur à ultrasons (136), pour produire des signaux ultrasonores pendant une phase d'émission au cours de laquelle l'amplificateur haute tension (132) fournit des signaux haute tension à sa sortie, et au moins l'un des transducteurs à ultrasons (112) étant conçu pour acquérir des signaux de mesure ultrasonores (140) ;
- au moins un préamplificateur basse tension (152) à faible bruit qui est disposé sur la deuxième zone (120), le préamplificateur basse tension (152) étant conçu pour recevoir le signal de mesure (148), le préamplificateur basse tension (152) comportant des dispositifs à semi-conducteur CMOS ;
- au moins un commutateur de sortie (150) qui est disposé sur la deuxième zone (120), le commutateur de sortie (150) étant conçu pour prévenir une sollicitation du préamplificateur basse tension (152) avec le signal de mesure (148) au moins pendant la phase d'émission ; et
- au moins une sortie de signaux basse tension (154) qui est disposée sur la deuxième zone (120) et qui est conçue pour transmettre un signal de mesure (148) préamplifié à une unité de commande et d'évaluation externe (212), **caractérisé par**
- au moins un multiplexeur (156) qui est conçu pour réunir des signaux de mesure ultrasonores (140), qui ont été acquis par au moins deux transducteurs à ultrasons (112) différents l'un de l'autre, en un signal de mesure commun (148),

une séparation (122) de la première zone (118) d'avec la deuxième zone (120) étant effectuée sur le substrat (116) au moyen de n puits, l'amplificateur haute tension (132) présentant au moins un amplificateur analogique linéaire à rétroaction (134) qui comporte au moins trois étages d'amplification (158, 160, 162) distincts, et l'amplificateur analogique linéaire à rétroaction (134) présentant une rigidité diélectrique d'au moins 80 V.

**2.** Dispositif (110) selon la revendication précédente, dans lequel la première zone (118) est conçue pour acquérir et traiter des tensions et des potentiels électriques de 5 V à 120 V et la deuxième zone (120) est conçue pour acquérir et traiter des tensions et des potentiels électriques de 1 nV à 10 mV.

**3.** Dispositif (110) selon l'une quelconque des revendications précédentes, dans lequel l'amplificateur haute tension (132) présente une première largeur de bande et dans lequel le préamplificateur basse tension (152) présente une deuxième largeur de bande, la première largeur de bande et la deuxième largeur de bande correspondant l'une à l'autre.

**4.** Dispositif (110) selon l'une quelconque des revendications précédentes, dans lequel chacun des étages d'amplification (158, 160, 162) comporte respectivement au moins deux transistors CMOS haute tension (M1, M2 ; M3, M4 ; M5, M6).

**5.** Dispositif (110) selon la revendication précédente, dans lequel un amplificateur différentiel (164) est présent à l'entrée d'un premier étage d'amplification (158), le premier étage d'amplification (158) et un deuxième étage d'amplification (160) étant respectivement disposés entre deux miroirs de courant (B, C), et dans lequel, dans le deuxième étage d'amplification (160) et dans un troisième étage d'amplification (162), les transistors CMOS haute tension (M3, M4 ;

M5, M6) sont respectivement reliés les uns aux autres.

6. Dispositif (110) selon l'une quelconque des revendications précédentes, dans lequel la première zone (118) présente en outre un commutateur d'entrée (130) qui est conçu pour permettre ou prévenir une sollicitation de l'amplificateur haute tension (132) par les signaux haute tension analogiques externes.

7. Dispositif (110) selon l'une quelconque des revendications précédentes, dans lequel la première zone (118) présente en outre un filtre réglable (142) qui est conçu pour supprimer des composantes de fréquence inférieures à une fréquence limite des signaux de mesure ultrasonores (140).

8. Dispositif (110) selon l'une quelconque des revendications précédentes, dans lequel le dispositif (110) se présente sous la forme d'un circuit intégré spécifique d'application (ASIC).

9. Écotomographe (200) pour examiner une partie corporelle, comprenant

   - un récipient (202) qui est conçu pour recevoir un objet à examiner et un milieu de couplage (210) ;
   - au moins un dispositif (110) selon l'une quelconque des revendications précédentes pour commander et lire un groupe de transducteurs à ultrasons (112) qui sont montés sur au moins une paroi du récipient (202) ; et
   - une unité de commande et d'évaluation (212) pour fournir des signaux haute tension analogiques destinés à au moins une entrée de signaux haute tension (128) du dispositif (110) et pour acquérir et évaluer des signaux de mesure ultrasonores qui sont fournis par au moins une sortie de signaux basse tension (154) du dispositif (110).

10. Écotomographe selon la revendication précédente, comprenant en outre une unité de sortie (216) qui est conçue pour afficher des images temps réel reconstruites.

## Fig. 1

## Fig. 2

Fig. 3a

Fig. 3b

Fig. 3c

## Fig. 3d

## Fig. 4

Fig. 5

Fig. 6

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2002030288 A1
- EP 2056124 A1
- WO 2011124379 A2
- WO 2012110228 A1
- WO 2005107962 A1
- US 20010043090 A1
- US 20090146695 A1
- US 20160242739 A1
- US 20150032002 A1
- WO 2017066564 A1
- KR 20160021354 A
- WO 2015186193 A1

**In der Beschreibung aufgeführte Nicht-Patentliteratur**

- **D.F. LEMMERHIRT** ; **X. CHENG** ; **R.D. WHITE** ; **C.A. RICH** ; **M. ZHANG** ; **J.B. FOWLKES** ; **O.D. KRIPFGANS**. A 32 × 32 Capacitive Micromachined Ultrasonic Transducer Array Manufactured in Standard CMOS. *IEEE Transactions on Ultrasonics, Ferroelectrics, and Frequency Control*, 2012, vol. 59 (7)
- **G. GURUN** ; **M.S. QURESHI** ; **M. BALANTEKIN** ; **R. GULDIKEN** ; **J. ZAHORIAN** ; **S.-Y. PENG** ; **A. BASU** ; **M. KARAMAN** ; **P. HASLER** ; **L. DEGERTEKIN**. Front-end CMOS Electronics for Monolithic Integration with CMUT Arrays: Circuit Design and Initial Experimental Results. *IEEE International Ultrasonics Symposium Proceedings (ULTSYM) 0096*, 2008
- **I. CICEK** ; **A. BOZKURT** ; **M. KARAMAN**. Design of a Front-End Integrated Circuit for 3D Acoustic Imaging Using 2D CMUT Arrays. *IEEE Transactions on Ultrasonics, Ferroelectrics, And Frequency Control*, 2005, vol. 52 (12)
- **J. SONG** ; **S. JUNG** ; **Y. KIM** ; **K. CHO** ; **B. KIM** ; **S. LEE** ; **J. NAB** ; **I. YANG** ; **O.-K. KWON** ; **D. KIM**. Reconfigurable 2D cMUT-ASIC Arrays for 3D Ultrasound Image. *Proc. of SPIE*, vol. 8320, 83201A-1
- **S.-J. JUNG et al.** Three-Side buttable integrated ultrasound chip with a 16x16 reconfigurable transceiver and capacitive micromachined ultrasonic transducer array for 3-D ultrasound imaging systems. *IEEE Trans. Electron Dev.*, 2013, vol. 60 (10), 3562-3569
- **R. WODNICKI**. Modular ultrasound arrays with co-integrated electronics. *Proc. of the 14th International Symposium on Nondestructive Characterization of Materials*, 2015, 23
- **H.-K. CHA**. CMOS ultrasonic analogue front-end with reconfigurable pulser/switch for medical imaging applications. *Electronics Letters*, 2015, vol. 51 (20), 1564-1566
- **H. KURUVEETTIL** ; **D. ZHAO** ; **C.J. HAO** ; **M. JE**. Analog Front End Low Noise Amplifier in 0.18-μm CMOS for Ultrasound Imaging Applications, International Journal of Electrical. *Computer, Energetic, Electronic and Communication Engineering*, 2013, vol. 7 (9)
- **K. CHEN** ; **H.-S. LEE** ; **A.P. CHANDRAKASAN** ; **C.G. SODINI**. Ultrasonic Imaging Transceiver Design for CMUT: A Three-Level 30-Vpp Pulse-Shaping Pulser With Improved Effciency and a Noise-Optimized Receiver. *IEEE Journal Solid-State Circuits*, 2013, vol. 48 (11)